# EUROPEAN PATENT APPLICATION

(11) **EP 4 796 629 A1**
(43) Date of publication of application: **26.08.2026**
(21) Application number: 25159886.8
(22) Date of filing: 25.02.2025
(51) Int. Cl.: C12N 1/20, A61K 35/744

(54) **PROBIOTIC BACTERIAL STRAINS AND COMPOSITIONS COMPRISING THEM**

(71) Applicant: Olimp Laboratories spolka z ograniczona Odpowiedzialnoscia, 39-200 Debica (PL)
(72) Inventor: JASTRZAB, Rafal, Krakow (PL); WIDLAK-KARGUL, Justyna, Nagawczyna (PL); MYTYCH, Jennifer, Rzeszow (PL)
(74) Representative: AOMB Polska Sp. z.o.o.

(57) **Abstract**

The present invention relates to four novel probiotic bacterial strains capable of direct manipulation of miRNA levels in a mammalian brain, as well as compositions for alleviating syndromes of neurodegenerative diseases, including in particular Alzheimer's disease (AD) and Parkinson's disease (PD) in a human patient, said composition comprising at least one probiotic bacterial strain. The strains and composition according to the present invention have an ability to improve cognitive performance in neuropsychiatric conditions, particularly neurodegenerative diseases, and show no risk of side effects in human patients, and thus are useful for oral administration (either as medicines or nutritional products) to alleviate symptoms of Alzheimer's and Parkinson's disease or dementia.

## Description

### Technical field

The present invention relates to four novel probiotic bacterial strains capable of direct manipulation of miRNA levels in a mammalian brain, as well as compositions for alleviating syndromes of neurodegenerative diseases, including in particular Alzheimer's disease (AD) and Parkinson's disease (PD) in a human patient, said composition comprising at least one probiotic bacterial strain.

### Background art

### Lactic acid bacteria as probiotics and postbiotics

Probiotics were defined as "live microorganisms that, when administered in adequate amounts, confer a health benefit on the host" (Hill et al., 2014). Multiple genera of bacteria are used as probiotics, however the major group which is recognized under this term is lactic acid bacteria. Lactic acid bacteria exist widely in nature and produce lactic acid during fermentation of carbohydrates. As main families which are widely distributed in various natural environments, *Lactobacillus* spp, *Lactococcus* spp and *Leuconostoc* spp can be mentioned. They are found not only in dairy food or in the intestines of humans and animals, but also in various vegetables, fruits and fermented food.

Postbiotics according to definition are "preparation of inanimate microorganisms and/or their components that confers a health benefit on the host". Postbiotics are deliberately inactivated microbial cells with or without metabolites or cell components that contribute to demonstrated health benefits. The beneficial effects of a postbiotic on health must be confirmed in the target host (species and subpopulation). The site of action for postbiotics is not limited to the gut (Salminen et al., 2021).

Both probiotics and postbiotics may exhibit health benefits for the host when ingested in adequate amounts (Costantini et al., 2017; Dikeocha et al., 2020; Jastrz 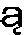 b et al., 2021; Nataraj et al., 2020; Saleena et al., 2023). Scientific literature indicates that lactic acid bacteria in mentioned form may be effective in alleviating symptoms and/or treating inflammatory bowel diseases, cancer, immune disorders, preventing gastrointestinal tract infections or improving digestion. However, this list is not restricted and probiotic (and gut microbiome) may positively modulate physiology of tissues and organs far beyond intestines - e.g. liver, lung, heart, brain *via* specific axis for example gut-brain-axis (Zhu et al., 2023).

### Probiotics and postbiotics in neurodegenerative diseases

Neurodegenerative disorders (ND) are a sub-group of neuropsychiatric conditions (diseases) that affect the neurons in the brain and spinal cord, leading to their degeneration and finally causing the loss of function in the affected areas. These disorders can be caused by a range of factors, including genetics, environmental factors, and lifestyle choices. At cellular level pathological signs are multifactorial and could be related to disrupted protein homeostasis (protein misfolding, proteasomal dysfunction, aggregation, inadequate degradation), imbalance in ox-redox equilibrium (increase in oxidative stress, free radical formation, mitochondrial dysfunctions) DNA damage, dysfunction of neurotrophins (NTFs) and dysregulated neuroinflammatory or neuroimmune processes.

Alzheimer's disease (AD) is the most common type of dementia. It is a progressive disease beginning with mild memory loss and possibly leading to loss of the ability to carry on a conversation and respond to the environment. AD involves parts of the brain that control thought, memory, and language (Mukherjee et al., 2018). Based on data from 2018 the number of patients with AD will triple to 14 million just in USA by 2060 (Matthews et al., 2019). To this date, there are studies suggesting that imbalances in gut microbiota can lead to neurological disorders through the gut-brain axis. People with gut disorders more prone to have AD in the future. Aging changes the gut microbial composition leading to overabundance of classified as pro-inflammatory taxa which deteriorates the permeability of the blood-brain barrier and GIT (Gastro Intestinal Tract) functions (Ghosh et al., 2022). For example, the presence of *Helicobacter pylori* in the gut microbiota increases the release of inflammatory mediators, which increases the amyloid β 40/42 ratio in the serum, other bacteria such as *Borrelia burgdorferi* and *Chlamydia pneumoniae* also participate in the hyper phosphorylation of tau protein which is hallmark of AD (Douros et al., 2024).

Parkinson's Disease (PD) is a neuronal disease that is characterized by tremors, slow movement, akinesia, muscular rigidity, gait, and difficulty in walking. Instead of these symptoms, PD patients suffer from constipation which is one of the causes of increased intestinal permeability and inflammation. These clinical manifestations are strongly related to changes in gut microbiome, whereas enrichment of the genera *Lactobacillus, Akkermansia,* and *Bifidobacterium* and depletion of bacteria belonging to the *Lachnospiraceae* family and the *Faecalibacterium* genus have been observed. Last two genera are important short-chain fatty acids producers, which reduction might result in a pro-inflammatory status which finally could be linked to the recurrent gastrointestinal symptoms affecting PD patients (Romano et al., 2021).Additionally, enrichment in *Escherichia coli* has been observed. This Gram-negative bacteria is capable to release amyloidogenic protein which induces alpha-synuclein aggregation and which regulates disease in the gut and neurodegeneration in the brain (Liang et al., 2023).

It has been proven that the inflammatory process and oxidative stress can be modulated by modifying the gut microbiota with the help of probiotics - in both mentioned diseases. Many *in vivo* and clinical trials have revealed that probiotics (*Lactobacillus acidophilus, Bifidobacterium bifidum,* and *Lactobacillus casei,* etc.) are effective candidates against the symptoms of AD (de Rijke et al., 2022; Mishra et al., 2024) and PD (Jin et al., 2024; Leta et al., 2021). Main limitation of mentioned studies was fact that they were performed mainly in chemically induced models of AD and PD *in vivo.* Both diseases are progressive and their onset in majority of cases are undetectable till cognitive or motor decline occur. To this date, there is no effective therapy registered and approved and many drugs (including probiotics) failed in different stages of drug discovery process (i.e. larger RCT) to prove their efficacy. This data suggests, that probiotics in proposed mechanism may act only in selected individuals and in specific moment of disease progression. Taken together there is an urgent need to find new strains, which biological mechanism of action is engaged in whole disease-lifespan and could dampen/slow down progression resulting in prophylactic alternative with effectiveness in larger group of patients.

### Summary of the invention

The inventive concept underlying the present invention is a new mechanism of action of selected probiotic strains, which - as it has been experimentally confirmed by the Inventors - directly modulate major signaling molecules - miRNA in affected brain structures. This concept is different and superior to approaches disclosed in the prior art due to fact that microRNA plays pivotal role in regulation of multiple molecular mechanism responsible for final cognitive and motor decline throughout all the stages of a neurodegenerative disease. MicroRNA (miRNA) are small, single-stranded, non-coding RNA molecules containing 21 to 23 nucleotides (DP Bartel, 2018). Found in plants, animals and some viruses, miRNAs are involved in RNA silencing and post-transcriptional regulation of gene expression (Bartel, 2004). miRNAs base-pair to complementary sequences in mRNA molecules, then silence said mRNA (Bartel, 2004; David P. Bartel, 2009). As a result miRNAs play a multiple roles in regulating cellular responses both in health and disease (Jonas & Izaurralde, 2015). For example, miRNA-107 and 31 are engaged in anti-inflammatory response during colitis (Xue et al., 2014).

It's well-known fact that microbiome is responsible for regulation of miRNA expression in various tissues including brain. For example, nude mice had reduced expression of miRNA107 in the hippocampus, whereas inoculation with the normal microbiota reversed this effect. The opposite relationship was observed for miRNA 32 (Chen et al., 2017). Despite that fact, there is no clear evidence in the prior art that specific genera, especially exogenously administered, may induce changes in miRNA profile.

There is some evidence that probiotics may manipulate levels of host miRNA - especially during inflammatory bowel diseases or colorectal cancer (Davoodvandi et al., 2021). Nevertheless, there is no data confirmed that probiotics may manipulate levels of miRNA directly in specific brain regions. Additionally, there is no evidence that a probiotic may be capable of manipulating miRNA-107, 31, 9, 181c and 376 and importantly this ability is genus-independent and may be common for different genera and selected strains.

The actual evidence available in the prior art that probiotic may be capable of manipulating host miRNA in different tissues including brain is sparse and provides no reliable basis for any more general considerations. At this moment relation of brain miRNA changes and probiotic administration has been established only for B. *longum* and notably this changes regarded mmu-mir-652-3p (DeVries et al., 2021). Due to tremendous genetic dissimilarity between *B*. *longum* and the bacterial strains according to the present invention as defined below it would have been impossible to reasonably assume that this effect might be common or similar. Additionally, there is no evidence whatsoever in the prior art that probiotics may be capable of manipulating mentioned miRNA (107, 31, 9, 181c) in brain and *via* that mechanism might improve cognitive abilities.

The present invention relates to a probiotic bacterial strain capable of direct manipulation of miRNA levels in a mammalian brain, said strain being selected from:
*Lactococcus lactis* OL3669, deposited with the KPD (Collection of Plasmids and Microorganisms at the University of Gdansk, Poland) under the accession number 1757,
*Lactiplantibacillus plantarum* OL3246, deposited with the KPD under the accession number 1760,
*Lacticaseibacillus paracasei* OL3110, deposited with the KPD under the accession number 1759, and
*Leuconostoc mesenteroides* OL3621, deposited with the KPD under the accession number 1758.

The probiotic bacterial strain defined above is useful in alleviating at least one syndrome of a neurodegenerative disease in a mammalian patient.

The present invention relates also to a composition for alleviating at least one syndrome of a neurodegenerative disease in a human patient, said composition comprising at least one probiotic bacterial strain defined above in an effective amount and/or a bacteria cell culture supernatant obtained from a culture of at least one probiotic bacterial strain defined above and/or at least one isolated component of the bacteria cell culture supernatant. The bacteria cell culture supernatant in one of preferred embodiments is a cell-free supernatant. Isolated components the bacteria cell culture supernatant include proteins, polysaccharides, small molecules ie. Fatty acids, indole metabolites, amino acids and its derivatives.

Preferably, the neurodegenerative disease is selected from Alzheimer's disease (AD), Parkinson's disease (PD) and other diseases negatively affecting neuronal system and being associated and/or modulated by miRNA 107, 31, 181, 376 and 9.

Preferably, said at least one probiotic bacterial strain defined above contains live bacteria, dead bacteria or both live and dead bacteria.

Preferably, the concentration of live or dead bacteria ranges from 10³ to 10¹¹ CFU per gram of dry weight of the composition.

Preferably, the bacteria of at least one probiotic bacterial strain defined in claim 1 and/or the bacteria cell-free supernatant and/or the isolated component thereof are in lyophilized form.

Preferably, said at least one probiotic bacterial strain defined above is form of a culture suspension.

Preferably, the composition according to the invention is in a solid form selected from capsule, tablet and powder or granulate for forming a solution or suspension.

In an alternative preferred embodiment, the composition according to the invention is in a liquid form selected from solution, suspension and emulsion. Preferably, the concentration of live or dead bacteria ranges from 10³ to 10¹¹ CFU per millilitre of the liquid composition. Further, preferably, the bacteria cell culture supernatant concentration in the liquid composition ranges from 2 to 35% v/v.

Preferably, the bacteria are contained in microcapsules. More preferably, the microcapsules contain live bacteria as well as auxiliary substances selected from lipids, phospholipids, proteins, polysaccharides or a mixture thereof.

Preferably, the composition according to the invention comprises at least one pharmaceutically acceptable excipient, aromatizing agent and/or carrier.

The bacteria strains of the present invention, i.e. *Lactiplantibacillus plantarum* OL3246 KPD1760, *Lacticaseibacillus paracasei* OL3110 KPD1759, *Leuconostoc mesenteroides* OL3621 KPD1758, *Lactococcus lactis* OL3669 KPD1757 are useful for manufacturing of probiotic compositions for oral administration to a human patient in need thereof. These compositions may take form of pharmaceutical formulations, functional food/nutraceuticals or dietary supplements. In such compositions the bacteria can be used in various forms, including live cultures, postbiotics in form of dead bacteria, post-cultivation supernatants (syn. Cell-free supernatants), extracts or lysates, as well as postbiotic fractions (isolates), and any combinations thereof.

### Brief description of figures

The present invention has been illustrated in exemplary embodiments at figures of drawings, showing:
- Fig. 1a: phylogenetic tree of 20 selected strains, providing a comparison of OL3110 strain of *Lacticaseibacillus paracasei* with the closest strains deposited in BV-BRC (PROKKA) database;
- Fig. 1b: circos plot representing protein similarity between OL3110 strain of *Lacticaseibacillus paracasei* and the two closest strains shown in Fig. 1a;
- Fig. 2a: phylogenetic tree of 20 selected strains, providing a comparison of OL3246 strain of *Lactiplantibacillus plantarum* with the closest strains deposited in BV-BRC (PROKKA) database;
- Fig. 2b: circos plot representing protein similarity between OL3246 strain of *Lactiplantibacillus plantarum* and the two closest strains shown in Fig. 2a;
- Fig. 3a: phylogenetic tree of 20 selected strains, providing a comparison of OL3621 strain of *Leuconostoc mesenteroides* with the closest strains deposited in BV-BRC (PROKKA) database;
- Fig. 3b: circos plot representing protein similarity between OL3621 strain of *Leuconostoc mesenteroides* and the two closest strains shown in Fig. 3a;
- Fig. 4a: phylogenetic tree of 20 selected strains, providing a comparison of OL3669 strain of *Lactococcus lactis* with the closest strains deposited in BV-BRC (PROKKA) database;
- Fig. 4b: circos plot representing protein similarity between OL3669 strain of *Lactococcus lactis* and the two closest strains shown in Fig. 4a;
- Fig. 5: heat-maps of changes of miRNA levels in HT-22 cells after treatment with probiotic strains according to the present invention and their postbiotics;
- Fig. 6a-6f: representation of miRNA changes after treatment with LB, HK, SNs and controls;
- Fig. 7: diagrams of changes of selected miRNA after treatment with strains according to the present invention in mice model of AD;
- Fig. 8: diagram of changes of selected miRNA after treatment with strains according to the present invention in mice model of PD;
- Fig. 9: open field results for specific parameters strains according to the present invention in mice model of AD;
- Fig. 10: open field results for specific parameters strains according to the present invention in mice model of PD;
- Fig. 11: Pearson's Correlation matrix between open field parameters and miRNA changes in AD mouse model;
- Fig. 12: Pearson's Correlation matrix between open field parameters and miRNA changes in PD mouse model;
- Fig. 13: Rota-rod test results;
- Fig. 14a-e: results of biochemical assays of previously described molecular markers associated with AD and PD.

### Detailed description of the invention

As already indicated above the present invention relates to four strains of novel lactic acid bacteria which belongs to *Lactobacillus, Lactoccocus* and *Leuconostocc* families. These bacteria were identified as: *Lactiplantibacillus plantarum* OL3246 KPD1760, *Lacticaseibacillus paracasei* OL3110 KPD1759, *Leuconostoc mesenteroides* OL3621 KPD1758, *Lactococcus lactis* OL3669 KPD1757. All were isolated and identified for the first time from natural sources.

The present invention relates to use of these strains for alleviating syndromes of neurodegenerative diseases, including in particular Alzheimer's disease (AD) and Parkinson's disease (PD) as well as other neuropsychiatric conditions which relate to disruption of regulatory miRNA in specific brain areas. In particular the novel bacteria strains are useful in manufacturing probiotic compositions for oral administration to human patients suffering from neurodegenerative disorders. Due to progressive character of neuropsychiatric conditions and miRNA disruption at all stages of disease, use of compositions which include the novel bacterial strains is not limited to any specific phase and also includes prophylactic use in individuals at early stage of such disease or even before a diagnosis - in case of individuals from high risk group (e.g. due to genetic predispositions to neurodegenerative diseases or advanced age).

### Technical background

Present invention describes four strains of lactic acid bacteria from different genera with common ability to alleviate cognitive and motor disabilities via modulation of miRNA in two brain regions - cerebellum and hippocampus. This effect is independent from anti-inflammatory activity or indirect activation of gut-brain axis (Experimental example 10), which was confirmed in *in vitro* and *in vivo* experiments. In both studies, described modulatory activity of probiotics and postbiotics are well correlated, which indicates direct interaction of molecules produced by certain strains with neurons.

Compared to the relatively closest prior art such as KR102523743B1 and KR1020200067299A, strain OL3669 is not identical when compared to SK-21, and isolated from vegetal source KC24 strain (strains from different environments are not closely related (Zhang & Cai, 2014)). Moreover, results provided in these prior art publications are restricted only to *in vitro* studies, which cannot be extrapolated to cognitive improvement in higher models. Importantly, in both publications the authors relied on different mechanism of action associated with improved neuronal survivability due to changes in anti-inflammatory response which in sum - is not associated with modulation of miRNA profile. Despite differences in methodologies, the strain OL3669 exhibited higher neuroprotection than SK-21 and has neutral influence on immunological profile *in vivo.*

Similar type of differences can be noted if strain OL3246 is compared to the relatively closest prior art. For example, in EP3747988A4, strain *L. plantarum* kbl396 was able to improve cognitive performance *in vivo via* modulation of serotoninergic pathways and upregulation of serotonin synthesis, which is a different mechanism, unrelated to manipulation of miRNA profile. Importantly, the experiment disclosed in EP3747988A4 has been performed on 7-week C57BL/6 mice without any neurodegeneration or chemically induced cognitive deficits, which is not a valid model of AD or PD. Further, in CN112915109A, CN110066753A and EP2937424A1, disclosing the use of strains *L. plantarum* DP189 and PS128, the authors revealed another mechanism of action which relied on reduction of inflammation (gliosis) and hyperactivation of glial cells in brain. This mechanism may be classified as anti-inflammatory action, which finally resulted in improvement in cognitive and motor abilities. When compared to the present invention, DP189 and PS128, are not closely related genetically with *L. plantarum* OL3246 so cannot be used as an prompting leading to the strains of the present invention. Additionally, the mechanisms described in these prior art publications are unrelated to modulation of miRNA profile, but instead are related to modification of immune profile and beta-amyloid deposition, which is not influenced by OL3246.

Another mechanism disclosed in the prior art relies on change in levels of brain neurotrophic factor (BDNF). This mode of action has been proposed for *Leuconostoc mesenteroides* strain (KCCM 12614P) and for *Lactobacillus casei* HY2782. Regarding KCCM 12614P, disclosed in KR1020220071167A, the authors did not confirm their hypothesis in any higher model than *in vitro* studies. Extrapolation of effectiveness from *in vitro* studies to higher models, including humans, is impossible in case of neurodegenerative diseases and is well described in scientific literature (Sun et al., 2022). In case of *Lactobacillus casei* HY2782, disclosed in KR102404658B1, the authors observed improvement in chemically induced model, which does not reflect a disease progression. Additionally, they declared that strain HY2782 is unique and not described before, based on standard 16s rDNA sequencing (partial sequence), which is commonly used to identify bacteria down to the genus and/or species level not strain (Johnson et al., 2019). This data is insufficient to claim that HY2782 is unique and there is no other strains with the same 16s rDNA sequence which pose similar properties. Moreover, there is no full genomic sequence in public databases, which could confirm that this strain is unique). HY2782 is genetically similar to KL-1-1 which full genome sequence is available. Following the comparison of entire genome of OL3110 and KL1-1 strains the present Inventors found that more than 60% of proteins are dissimilar, which clearly indicates the unique character of OL3110. Finally, the authors KR102404658B1 declared that HY2782 strain is able to improve cognitive performance via upregulation of BDNF and downregulation of beta-amyloid 42 deposition. In the studies performed using OL3110 strain the present Inventors did not observe any changes in levels of beta-amyloid and BDNF in tested animals - both in brain and in blood (Experimental example 10). This data pinpoints that strain OL3110 have different mechanism of action than HY2782. Taken together, the disclosure of KR102404658B1 and KR1020220071167A provides no meaningful input information about possible positive influence of OL3110 and OL3621 on cognitive abilities, *via* modulation of miRNA profiles directly in specific brain structures.

Importantly, there is substantial progress in establishing genetic-probiotic relationship of gut bacteria including lactic acid bacteria strains. A few years ago, positive probiotic features were transferred between all representatives of genus. At this moment it is a known fact that health beneficial properties of bacteria are strain-dependent. Recent studies have shown that genetically and phenotypically bacteria strains of the same genus can differ as much as humans and lemurs (Garcia-Gonzalez et al., 2022; Jäger et al., 2019). The overall conclusion could be made that even if the strains are very closely related, there is no guarantee that biological properties will be the same or even similar. This fact highlights the utmost importance of individual screening and inability to use the current state of knowledge/technology as a basis or indicator of possible health-promoting effects. Moreover, the lack of effective therapies for neurodegenerative diseases creates an urgent need to search for new, effective options for alleviating cognitive deficits.

The present invention presents a unique approach to screening and phenotyping of lactic acid bacteria, unrelated to the prior art discussed above. This approach resulted in selection of four strains from different genera. The selected strains are capable of alleviating cognitive deficits *via* common (new) mechanism of action, i.e. the restoration of miRNA levels in affected brain structures in neurodegenerative disorders, including in particular the two different neurological diseases, namely AD and PD.

Problems solved by the present invention include:
- ameliorating cognitive deterioration and symptoms of neuropsychiatric conditions via manipulation of miRNA signaling pathways by the novel probiotic strains - as the main mechanism of action;
- declining the onset of cognitive and motor disabilities associated with progression of neurodegenerative diseases *via* direct manipulation of signaling miRNA in affected brain structures by unique probiotic strains and their constituents.

The results of *in vitro* and *in vivo* experiments described and discussed in detail in the Examples below provide evidence of influence of the four novel probiotic strains on regulatory miRNA, leading to solving the problems indicated above. The Examples confirm the unique character of isolated strains, show the influence of these strains on regulatory miRNA examined both *in vitro* and *in vivo, and also show the* correlation between miRNA regulation and cognitive/motor abilities.

The probiotic formulations containing bacterial strains according to the present invention include in particular oral dosage forms such as capsules, tablets and powder for preparing aqueous suspensions.

In case of capsules or tablets with strain(s) in form of lyophilized powder without additives, the active substances preferably include at least one bacterial strain according to the invention in the form of lyophilized/microencapsulated bacteria (OL3669, OL3621, OL3246 or OL3110, or a mixture thereof) in concentration 10^11 CFU/g (the strain can be present in a live or dead form (heat-killed as described before); in case of a dead form, the CFU/g quantifies viable bacteria before the killing process], constituting 1%-40% of the capsule filling/tablet mass. Typical excipients include filling substances, such as cellulose and/or microcrystalline cellulose and/or starch and/or pregelatinized starch and/or maltodextrin and/or lactose and/or calcium hydrogen phosphate and/or inactivated yeast in an amount ranging from 50 to 85% by weight; glidants such as magnesium salts of fatty acids and/or calcium salts of fatty acids and/or talc in an amount ranging from 0.5 to 5% by weight; anticaking agents such as silica, colloidal silica in an amount ranging from 0.1 to 2% by weight. Capsule shells are typically made of cellulose, pullulan or gelatin, and preferably are enteric-coated. Total mass of capsule filling is typically 500 mg.

In case of capsules with strain(s) in form of lyophilized powder with additives, the active substances in addition to bacterial strains mentioned above may also include vitamins e.g. vitamin B6 and minerals e.g. magnesium salts in an amount of 0,5%-15% by weight, as well as herbal extracts, amino acids and proteins in an amount of 0,5%-15% by weight. Typical excipients include filling substances, glidants and anticaking agents as mentioned above, but the typical amount of filling substances ranges here from 25 to 85% by weight. Typical capsule shell materials and typical capsule filling mass are the same as above.

Further, in case of powder for preparing aqueous suspensions, the active substances preferably include at least one bacterial strain according to the invention in the form of lyophilized/microencapsulated bacteria (OL3669, OL3621, OL3246 or OL3110, or a mixture thereof) in concentration 10^11 CFU/g (the strain can be present in a live or dead form (heat-killed as described before); in case of a dead form, the CFU/g quantifies viable bacteria before the killing process], constituting 1%-10% of the powder mass. Typical excipients include filling substances, such as maltose and/or maltodextrin and/or sorbitol and/or xylitol and/or isomalt and/or starch and/or glucose and/or fructose in an amount ranging from 89,7% to 95,5% by weight; acidity regulators such as citric acid and/or malic acid, in an amount ranging from 0.1 to 1% by weight; anticaking agents such as silica, colloidal silica in an amount ranging from 0.1 to 0.5% by weight; and flavoring substances in an amount ranging from 0.1 to 1% by weight. Typical mass of a single dose of powder is 5 g.

In case of powder with strain(s) in lyophilized form with additives the active substances in addition to bacterial strains mentioned above may also include vitamins e.g. vitamin B6 and minerals e.g. magnesium salts in an amount of 0,5%-25% by weight, as well as herbal extracts, amino acids and proteins in an amount of 0,5%-55% by weight. Typical excipients include filling substances, acidity regulators, anticaking agents and flavoring substances as mentioned above, but the typical amount of filling substances ranges here from 39.7% to 95.5% by weight. Typical mass of a single dose of powder in such case is 10 g.

Finally, in case of a cell-free supernatant solution in liquid form with additives, the active substances typically include a cell-free supernatant obtained from at least one bacterial strain according to the present invention (OL3669 or OL3621 or OL3246 or OL3110 or a mixture thereof) in an amount ranging from 1% to 35% (v/v); vitamins e.g. vitamin B6 and minerals e.g. magnesium salts in an amount of 0,5%-25% (w/v); and herbal extracts, amino acids and proteins in an amount of 0,5%-25% (w/v). Typical excipients include water in amount of 65%-99% (v/v); acidity regulators e.g. citric acid and/or malic acid in an amount of 0.1-1% (w/v); flavoring substances in an amount of 0.1-1% (w/v); and preservatives in an amount of 0.1-1% (w/v). Typical single dose of such solution is 50 ml.

Preparation of oral dosage forms described above generally involves the following general steps:

### 1. Preparation of the environment

Provide appropriate air temperature and humidity conditions for weighing preparation and direct packaging. Minimum requirements include temperature below 25°C and humidity below 35%. However, optimal conditions are achieved at the air temperature of 20-22°C and humidity not exceeding 20%. Optimal conditions are maintained through proper air flow regulation combined with an appropriate ventilation system using air conditioners and dehumidifiers. The process area is subject to cleaning procedures using properly selected washing and disinfecting agents. Appropriate protective gear is provided for staff working with biological materials and composition ingredients, including a gown, pants, shoes, cap, mask, goggles, and shoe covers.

### 2. Preparation of raw materials

Use pre-tested and quality-approved raw materials appropriate for the given formulation. Condition biological material for a minimum of 2 hours before weighing.
3. Weighing of raw materials
4. Mixing or dissolving the obtained raw materials
5. Capsule/Sachet/bottle filling or Tableting

### Examples

The following examples are the experimental confirmation of unique character and properties of the tested strains.

### Experimental example 1 - Reciprocal blast analysis

Strains were isolated from natural sources (see Table 1 below) and isolated DNA were subjected to whole genome sequencing as described by Salanski et al., 2022 to verify uniqueness of tested bacteria. In the next step, a bioinformatic analysis was performed to compare genomes of OL3110, OL3246, OL3621, OL3669 strains with their closest neighbors. Using widely recognized and open access tools in BV-BRC database, which is the biggest curated and validated library of procaryotic genomes (Olson et al., 2023), the present Inventors selected 20 strains closest to each of four novel bacteria strains according to the invention. The selection criterium was the lowest Jaccard distance and the highest values of k-mers in Mash/MinHash analysis (Ondov et al., 2016). The results were used to create phylogenetic trees, using phylogenetic tree service (BV-BRC Codon Tree webtool).

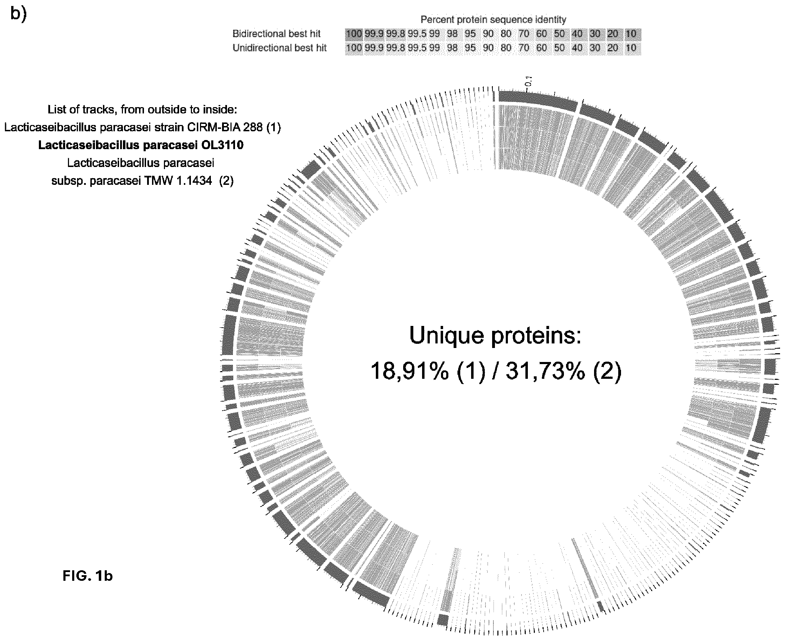

Based on the obtained results, two closest strains for each novel bacteria strain of the invention were selected and subjected to reciprocal proteome comparative analysis. The BV-BRC Proteome Comparison service is based on the original Sequence-based Comparison tool that was part of RAST (Aziz et al., 2008). This tool colors each gene based on protein similarity using BLASTP and marks each gene as either unique, a unidirectional best hit or a bidirectional best hit when compared to the reference genome. The output includes a whole-genome schematic that is colored based on BLAST. Due to the fact that proteome is mainly responsible for phenotypic differences between closely related strains of bacteria, the present Inventors compared proteomes to finally prove that four selected bacteria strains are novel and had not been described before. All the relevant information is presented on Figs. 1b, 2b, 3b, 4b. The cutoff of unique proteins was selected as percentage of identity < 1.

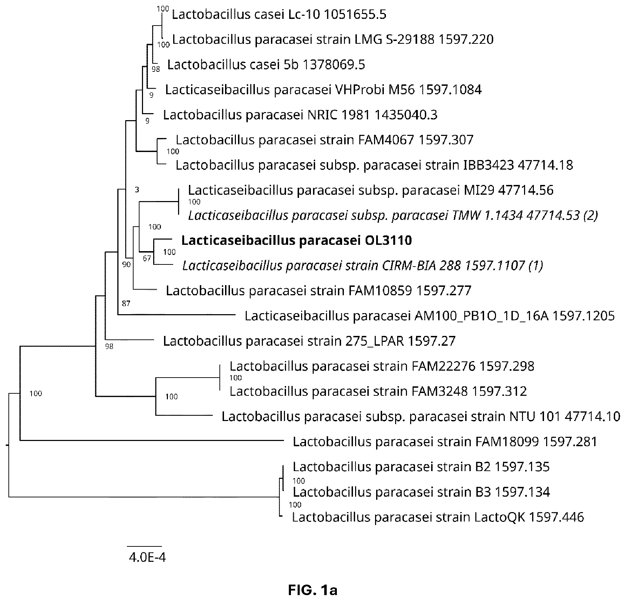

Fig. 1a and 1b show phylogenetic and proteomic comparison of OL3110 strain of *Lacticaseibacillus paracasei,* with the closest strains deposited in BV-BRC (PROKKA) database. More specifically, fig. 1a shows a phylogenetic tree of 20 selected strains. Strains were selected based on Mash/MinHash results of Jaccard distances and number of common k-mers. Strain of interest (OL3110) is highlighted by bold font; strains used for proteome comparison - in italics. In turn, fig. 1b show circos as a graphical representation of protein similarity between two closest selected strains. The legend above the circular diagram indicates percentage of difference. Description on left side defines the track order.

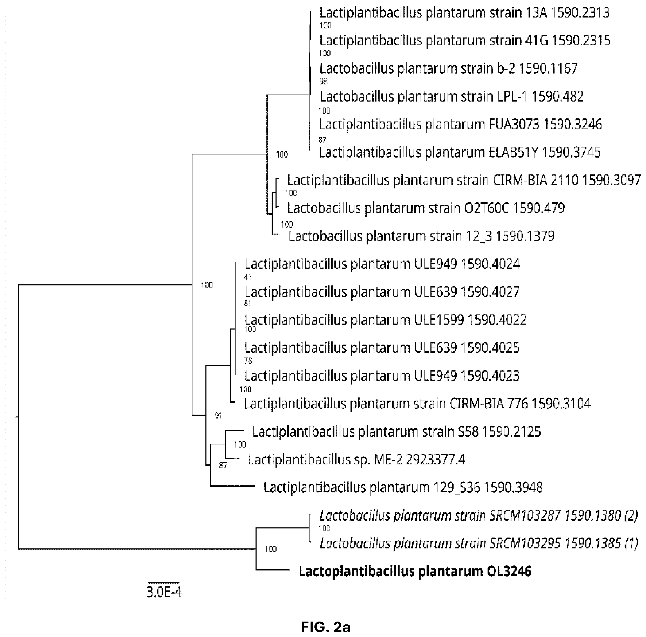

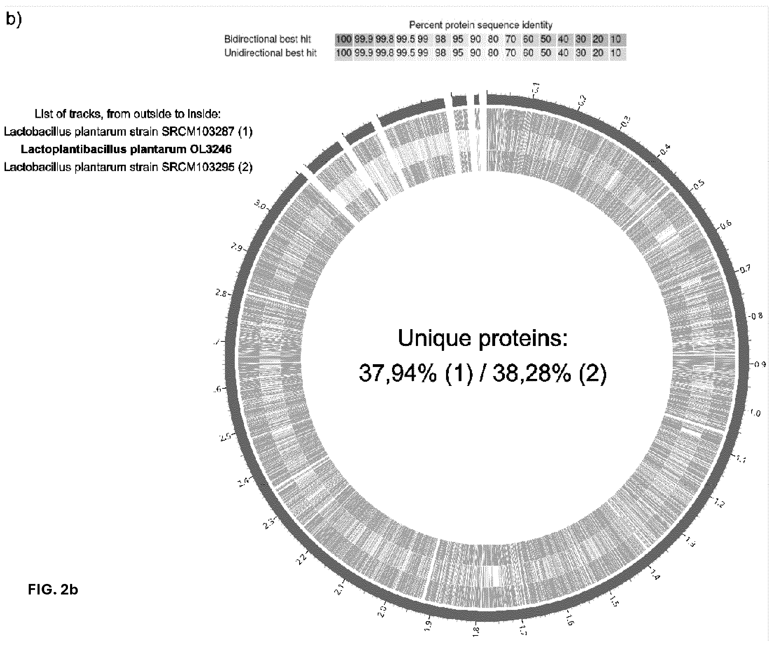

Figs. 2a and 2b show phylogenetic and proteomic comparison of OL3246 strain of *Lactiplantibacillus plantarum,* with the closest strains deposited in BV-BRC (PROKKA) database. More specifically, fig. 2a shows a phylogenetic tree of 20 selected strains. Strains were selected based on Mash/MinHash results of Jaccard distances and number of common k-mers. Strain of interest (OL3246) is highlighted by bold font; strains used for proteome comparison - in italics. In turn, fig.2b show circos as a graphical representation of protein similarity between two the closest selected strains. Legend above circular diagram indicates percentage of difference. Description on left side define the track order.

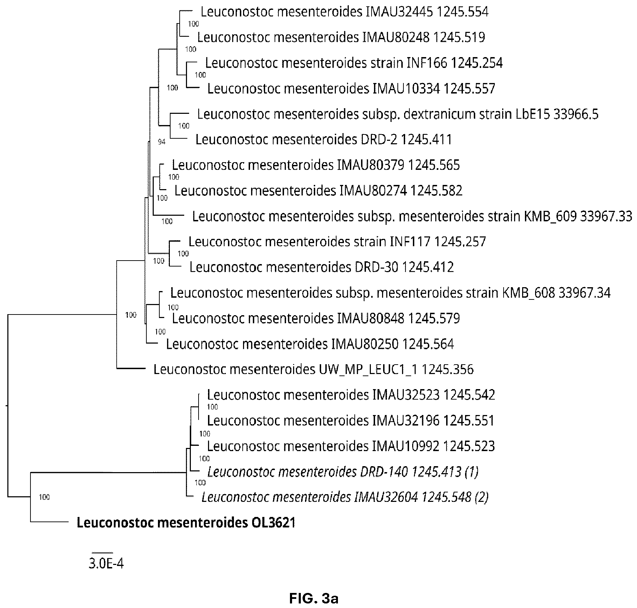

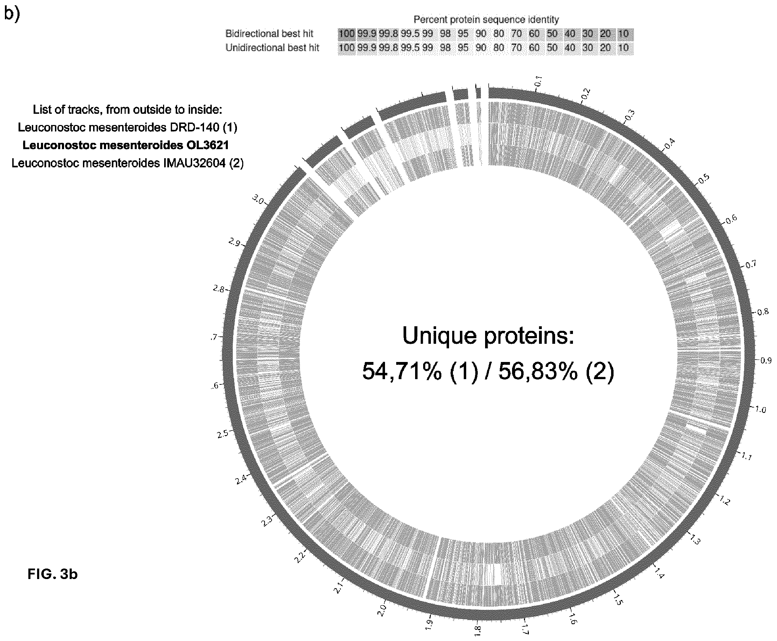

Figs. 3a and 3b show phylogenetic and proteomic comparison of OL3621 strain of Leuconostoc mesenteroides, with the closest strains deposited in BV-BRC (PROKKA) database. More specifically, fig. 3a shows a phylogenetic tree of 20 selected strains. Strains were selected based on Mash/MinHash results of Jaccard distances and number of common k-mers. Strain of interest (OL3621) is highlighted by bold font; strains used for proteome comparison - in italics. In turn, fig. 3b show circos as a graphical representation of protein similarity between two the closest selected strains. Legend above circular diagram indicates percentage of difference. Description on left side define the track order.

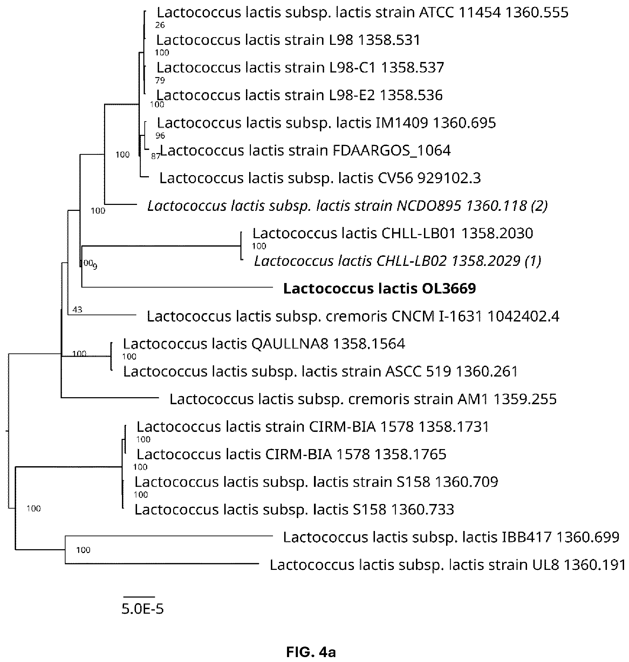

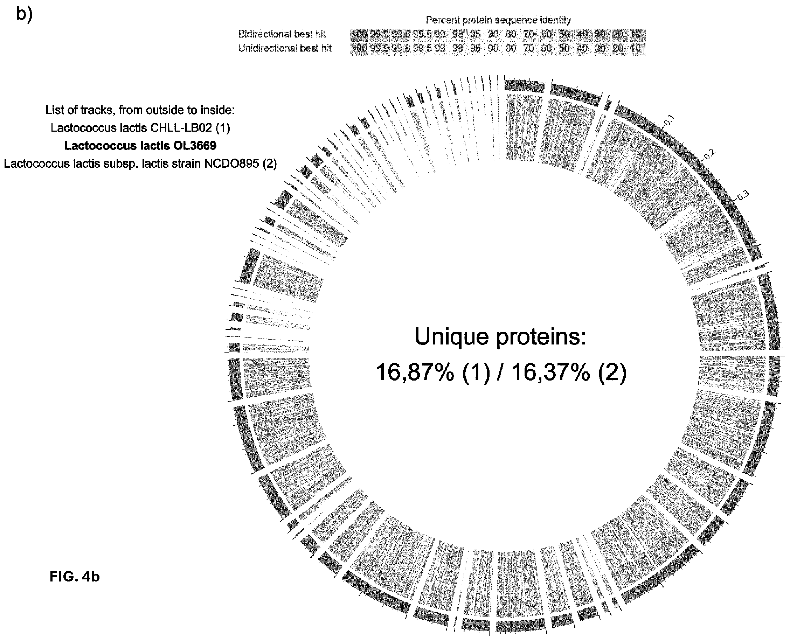

Figs 4a and 4b show phylogenetic and proteomic comparison of OL3669 strain of Lactococcus lactis, with the closest strains deposited in BV-BRC (PROKKA) database. More specifically fig. 4a shows a phylogenetic tree of 20 selected strains. Strains were selected based on Mash/MinHash results of Jaccard distances and number of common k-mers. Strain of interest (OL3669) is highlighted by bold font; strains used for proteome comparison - in italics. In turn, Fig. 4b shows circos as a graphical representation of protein similarity between two the closest selected strains. Legend above circular diagram indicates percentage of difference. Description on left side define the track order.

### Experimental example 2 - comparison of differences of metabolic properties of tested novel strains

Despite clear differences in genome and proteome of selected strains, the phenotypic features (commonly known as probiotic features), were evaluated. Among them, adhesion to mucins, bile and acid resistance, and metabolic potential can be mentioned. These data indicate differences in probiotic properties between strains of interest and due to wide range cannot be directly associated with final therapeutic effect. The data were collected in table 1.

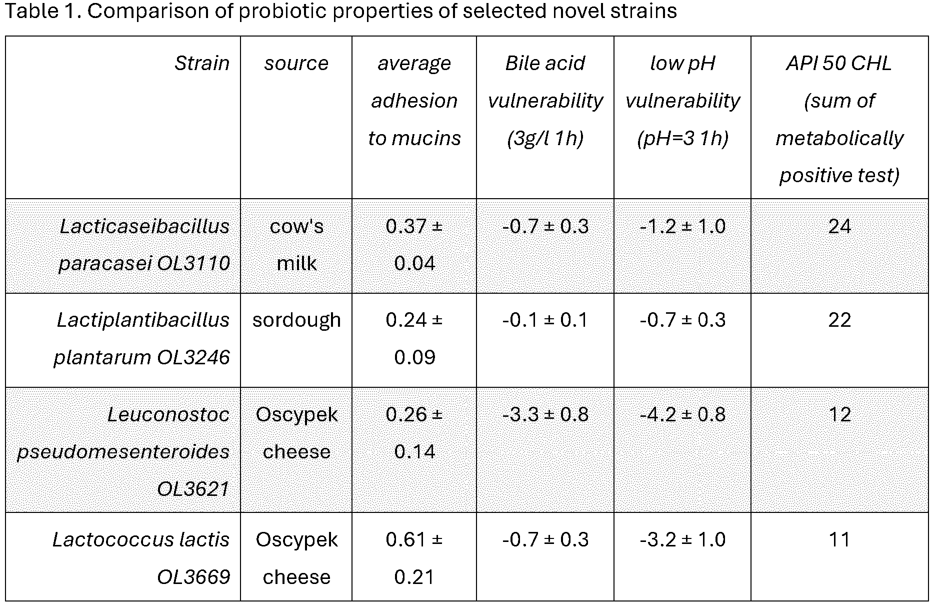

Average adhesion to mucins was tested as described in (Radziwill-Bienkowska et al., 2016). Adhesion values should be interpreted in comparison to indicator strains: TIL448 (strong adherence - 0.54), IBB477 (medium adherence - 0.27), IL1403 (no adherence - 0.18). Bile and acid resistance were presented as change in bacterial viability in agar plate method after 1h of exposure (Chopin et al., 1984; Le et al., 2013; Radziwill-Bienkowska et al., 2016). API50 CHL column contains sum of positive API test performed strictly under API 50 CHL producer recommendations (Biomerieux, France).

### Experimental example 3 - miRNA profile in vitro

As initial experiment, the present Inventors performed wide screening of influence of tested strains on miRNA profile in HT22 hippocampal cells treated with neurodegenerative agent - glutamate. This protocol is widely recognized and used to test effectiveness of new drug candidates and biology of aging, cellular stress and neurodegeneration (Prasansuklab et al., 2023; Sanderson et al., 2015).

Screening was divided into two steps. At first an assessment of toxicity of tested strains as live bacteria and postbiotic fractions (heat-killed and supernatants) was performed in MTS assay. This assay is designed to evaluate the metabolic activity and viability of cells. The assay involves the conversion of the tetrazolium salt MTS (3-(4,5-dimethylthiazol-2-yl)-5-(3-carboxymethoxyphenyl)-2-(4-sulfophenyl)-2H-tetrazolium) to a purple formazan in the presence of phenazine methosulfate. Based on obtained results, concentrations were selected and tested in miRNA assay (see the section 'Material and methods - Experimental example 3' below). The present Inventors decided to evaluate activity in simultaneous action - glutamate and test probiotic/postbiotics were added in the same time. This approach simulates the state of initiation and progression of neurotoxicity mechanism *in vitro.*

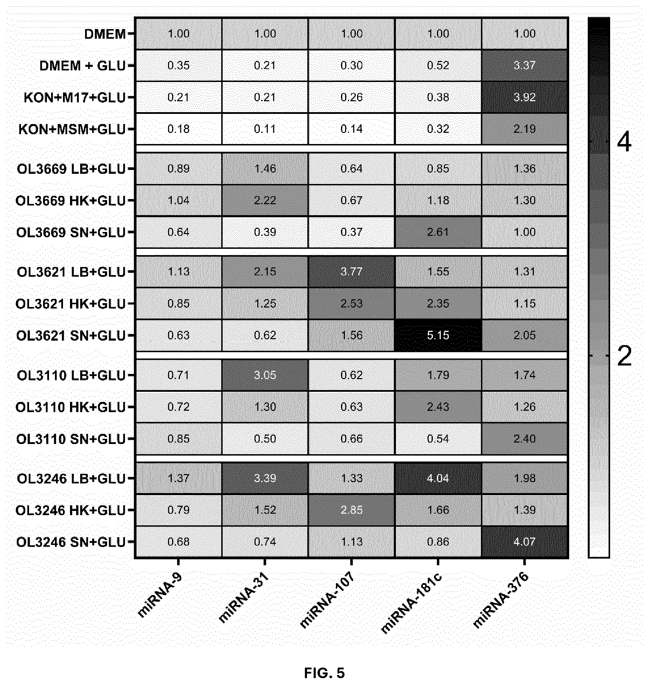

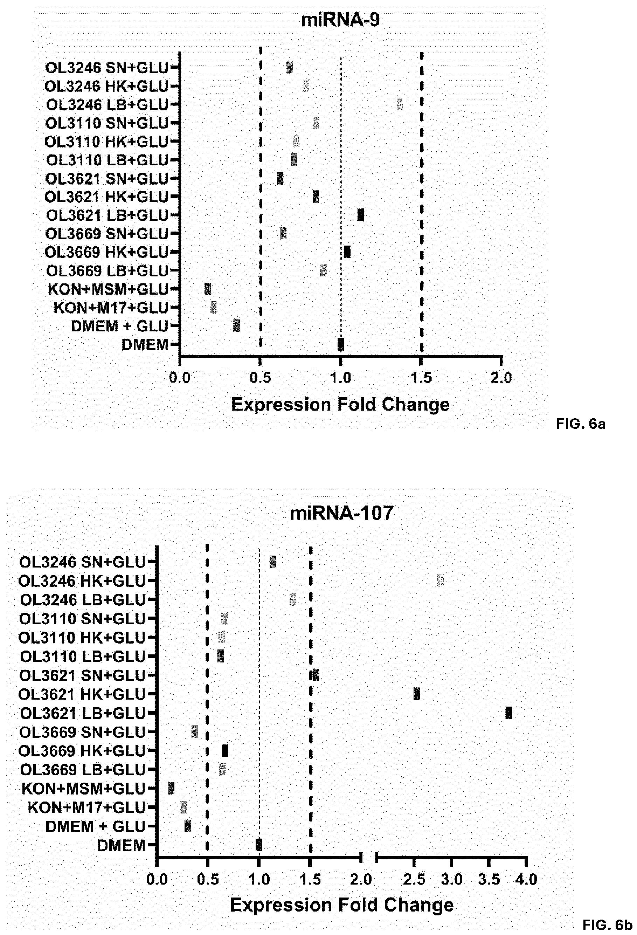

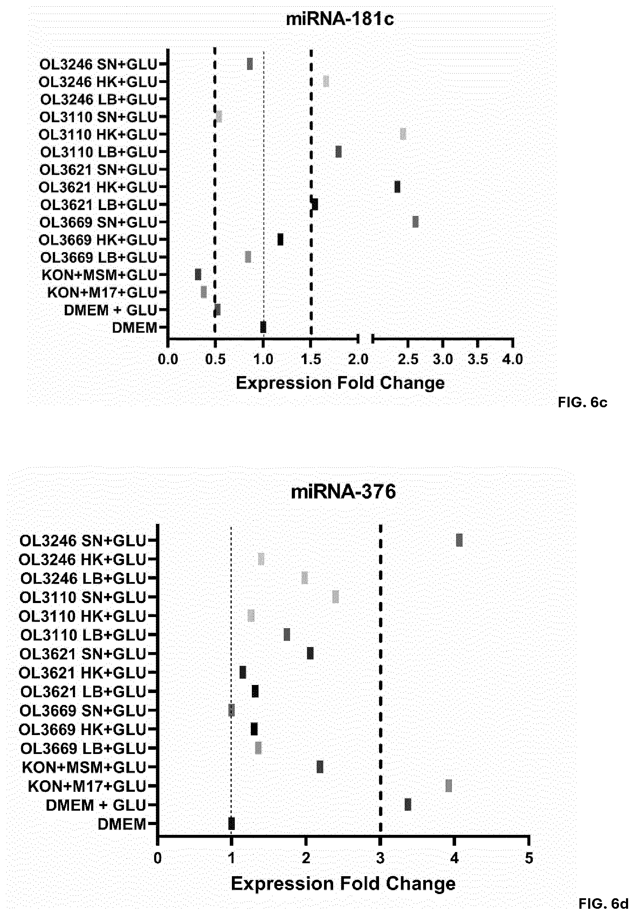

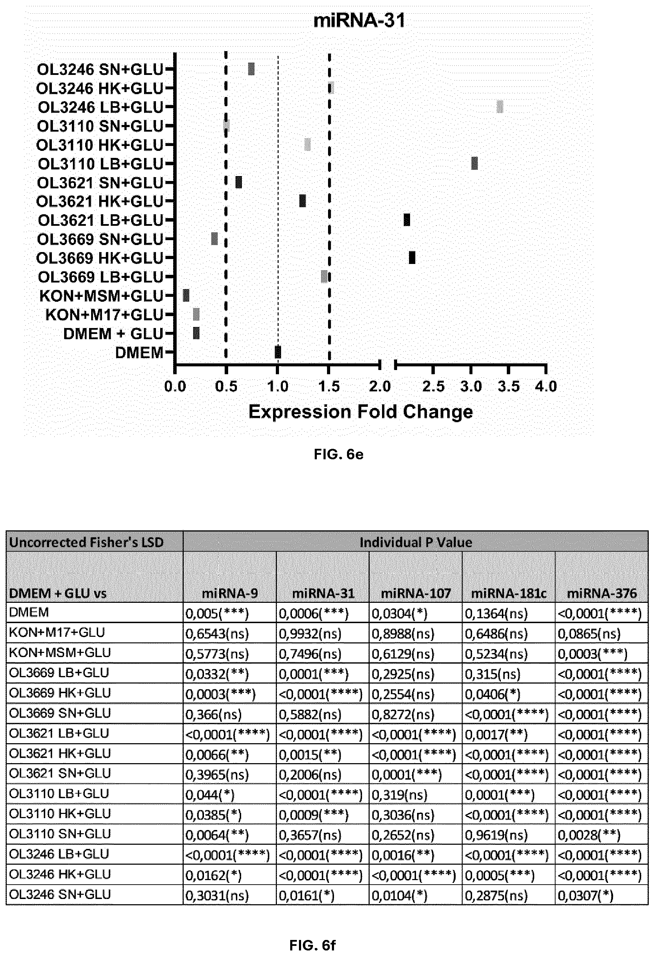

Initially 10 different miRNAs were tested, which have associations with neurodegeneration (Wei et al., 2020). Finally, significant positive regulation of 5 selected miRNA's was observed. The results are presented in Fig. 5 and Figs. 6a-6f and these miRNA molecules were selected for further *in vivo* experiment and final confirmation of positive effect on cognitive abilities. Amelioration of negative impact of L-glutamate on HT-22 cells was observed for all tested strains (Fig. 5), with differences in level of activity between live bacteria (LB) and postbiotic fractions (HK - heat-killed bacteria, SN - cell free supernatants) (Figs 6a-6f). In general, all postbiotic fractions and live bacteria positively modulate selected miRNA profile/restored normal miRNA profile.

Fig5 shows a heat-maps of changes of miRNA levels in HT-22 cells after treatment with unique probiotic strains and their postbiotics. Change of miRNA profile after treatment with live bacteria (LB -10³ bacteria/ml), heat-killed bacteria (HK-10⁵ bacteria/ml), supernatants (SN - 5%v/v) of each strain, which unique identifier/number is a prefix of particular treatment; suffix: +GLU - represents addition of 2.5mM of L-glutamate. DMEM is a control group without any treatment; KON+MSM - HT-22 cells with 5% (v/v) addition of designed *Lactobacilli* broth; KON+M17 - HT-22 cells with M17 + 0.5% glucose (w/v) added in 5% of final concentration. Both interventions were controls for SN treatments.

Figs 6a-6f show detailed representation of miRNA changes after treatment with LB, HK, SNs and controls. Each graph represents an expression fold change calculated as ratio between interventions and control (DMEM). Dashed lines represent no change in expression (1.0 - expression fold change), lowered expression (0.5), increased expression (1.5). In case of miRNA-376, there is two dashed lines due to lack of lowered expression. Labels of interventions, control and treatment are the same as on fig. 5. In overall, all tested interventions reversed negative impact of Glutamate on expression of tested miRNA in various degree. Some of tested interventions have directly opposite effect than glutamate (ie. OL 3669 HK on miRNA-31 expression); in other the magnitude of improvement were smaller (ie. OL 3669 SN on miRNA-31 expression), but still better than Controls (KON+M17+GLU, KON+MSM+GLU, DMEM+GLU), which proves positive influence of selected strains - both as probiotics and postbiotics, on miRNA profile. Statistical significance data were collected in table on fig. 6f.

### Material and methods - experimental example 3

### Culture conditions

The strains OL3621, OL3110 and OL3246 were cultivated in 37 °C, 24h at anaerobic conditions to simulate temperature and oxygen availability in the gut. Using MRS broth with 2% (w/v) addition of glucose.

The *Lactococcus lactis* OL3669 was cultivated during 24h in laboratory medium M17 with 0.5% (w/v) addition of glucose under the same conditions of temperature and oxygen availability as OL3621, OL3110 and OL3246.

### Preparation of heat-killed (HKB) and live bacteria (LB)

Based on the results of thiazole orange and propidium iodine staining (TO-IP), live and dead bacteria were counted in culture, as described in Davey & Kell, 1996. Next, sufficient volumes of bacterial suspension were transferred to two sets of separate tubes, where final bacterial concentration reached 10³, 10⁴,10⁵,10⁶, 10⁷, 10⁸ and 10⁹ live/ml each. Then one set of tubes was centrifuged (10min, 10 000 rpm, 4°C) and stored in fridge until experiment and the second set - was heat-killed in water bath (65°C, 35min) and centrifuged as described above. Heat killing process was confirmed by second run of TO-IP staining and flow cytometry. Dilutions of bacteria for miRNA assay were chosen based on preliminary screening.

### Preparation of supernatants

Supernatants obtained from centrifugation of bacterial suspensions were sterile filtered (0.2µm syringe filter, VWR), neutralized by addition of 1M of NaOH and stored prior to experiments at 4°C. The dilution suitable for miRNA was selected based on MTS results - similar as in case of HKB I LB.

### Cell cultures and conditions

Cells were cultured in a humidified incubator with 5% CO₂ at 37 °C. The HT22 (Merck, Cat. # SCC129) mouse hippocampal cell lines were grown in DMEM medium (VWR, cat. no. C392-0417) supplemented with 10% (v/v) fetal bovine serum (EurX, cat. no. E5050), without antibiotics.

### miRNA assay

Prior to experiment HT-22 cells were seeded for 24h at density 9375 cells/cm² (1.46 x 10⁶ cells/ml) in T25 bottles (extrapolated density from MTS assay to T25 bottle). Next, they were treated by selected concentration of LB (10³ bacteria/ml), HKB (10⁵ bacteria/ml), CFS (5% v/v) for 24h with and without 2.5 mM of L-glutamate. Then, medium was discarded, cells were washed twice with Hank's balanced salt saline and subjected to miRNA isolation based on manufacturer's recommendation of PureLink^{™} miRNA Isolation Kit (Invitrogen, K157001). Concentration and purity were verified by checking absorbance 260nm and 280nm using microplate reader (Thermo Fisher Scientific, CA, USA, Varioscan Lux). Based on concentration and purity results and recommendations of manufacturer of TagMan^{®} Advanced miRNA cDNA Synthesis Kit (#A28007, Applied Biosystems) miRNA was transcripted to cDNA. Then concentrations were evaluated, cDNA was 5x diluted and used for PCR reaction according to manufacturer's recommendations (TaqMan^{™} Fast Advanced Master Mix, TaqMan^{™} Advanced miRNA Assay), using Azure Biosystems RT-PCR thermocycler. Results were analyzed with ΔΔCt method using untreated cells as baseline (DMEM group) to calculate fold change. Statistical analysis between groups was performed using ordinary one-way ANOVA without correction for multiple comparisons (Fisher LSD test).

### Experimental example 4 - miRNA profile in vivo

Observed *in vitro* results suggested that probiotics and postbiotics of selected strains have the ability to reduce neurodegeneration (as a result of restoration of miRNA profile and improvement of viability in MTS assay). To finally confirm that modulation of miRNA may have positive influence on cognitive abilities, the selected strains were tested in *in vivo* models of AD and PD. Due to fact that both diseases are progressive, genetic models were used. Tg2576 (Taconic Biosciences) was used as the AD model (Heiland et al., 2019; Westerman et al., 2002), which simulates neurodegeneration via accumulation of misfolded beta-amyloid proteins. For Parkinson's disease progressive the model of alpha-synuclein accumulation A53T (Jackson's Laboratory) (Paumier et al., 2013) was chosen.

Mice were fed during 60 days once a day with lyophilized suspension of selected strains and, at the end of trial, cognitive and motor test has been performed (open field test, Rota-rod test), then mice have been euthanized and miRNA levels in hippocampus (AD) and cerebellum (PD) were determined (see 'Experimental example 4 - material and methods' below).

The Inventors observed similar pattern of downregulation of miRNA-9, miRNA-31, miRNA-107, miRNA-181c in control AD-mice, as in *in vitro* experiments with glutamate-induced neurotoxicity. Additionally, the healthy control (ATG) had higher levels of mentioned miRNAs and all four interventions restored levels of selected miRNAs or made it closer to non-genetically modified controls (AK). In case of miRNA-376 which is associated with PD, but also dysregulated in *in vitro* model, positive association in both models (AD and PD) was observed. PD control animals had higher levels of miRNA-376 in cerebellum, similar to HT-22 cells treated with 2,5mM of L-glutamate. Importantly, administration of all four strains to PD-mice downregulated expression to similar levels as in healthy animals (PTG). Taken together a positive correlation was obtained between two experimental models *in vitro* and *in vivo,* allowing to conclude that the modulation of miRNA is responsible for positive cognitive effects, which were observed in open field test and Rota-rod (see 'Experimental example 5' below).

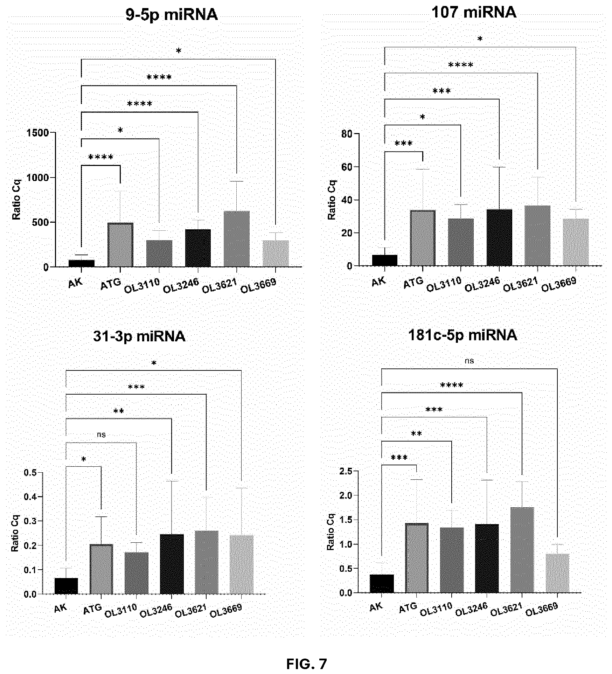

Fig. 7 show a diagram of changes of selected miRNA after treatment with strains in mice model of AD. Presented miRNA was selected in miRNA-array screening and further confirmed and quantified using qRT-PCR method. All analyses were performed on brain tissue samples (hippocampus). Each plot represents data for selected miRNA (described above). In this experiment, genetic background (ATG) was used as a healthy control, genetically modified mice (AK) as a control with disease. Statistical analysis was performed using ordinary one-way ANOVA with Dunnett correction against AK group. (ns - statistically insignificant (P ≥ 0.05); * - statistically significant (P ranging from 0.01 to 0.05); ** - statistically significant (P ranging from 0.001 to 0.01); *** - statistically significant (P < 0.001); **** - statistically significant (P < 0.0001).

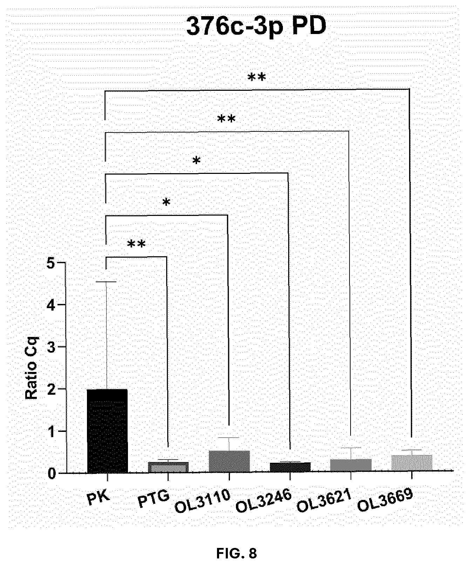

Fig 8 shows a diagram of changes of selected miRNA after treatment with strains in mice model of PD. Presented miRNA was selected in *in vitro* miRNA screening described above and further confirmed and quantified using qRT-PCR *in vivo* method. All analyses were performed on brain tissue samples (cerebellum). Each plot represents data for selected miRNA (described above). In this experiment genetic background (PTG), was used as a healthy control, genetically modified mice (PK) as a control with disease. Statistical analysis was performed using ordinary one-way ANOVA with Dunnett correction against AK group. (ns - statistically insignificant (P ≥ 0.05); * - statistically significant (P ranging from 0.01 to 0.05); ** - statistically significant (P ranging from 0.001 to 0.01); *** - statistically significant (P < 0.001); **** - statistically significant (P < 0.0001).

### Material and methods (Experimental example 4)

### Animals - housing and handling

The experiment used mice of the B6;SJL-Tg(APPSWE)2576Kha line (#1349-M Taconic Bioscience), which is a model of Alzheimer's disease (mutants), and the B6SJLF1/J line (Stock No: 100012 The Jackson Laboratory),which is a control group for mutants (hereinafter called genetic background). Animals were housed in groups of 3-5 animals per cage and separated by sex with food and water *ad libitum* at 23 °C for 12 hr day/12 hr night cycles in cages that contained plastic houses and shredded paper flakes to allow nest building. All experimental protocols were approved by appropriate local bioethics commission and all methods were carried out in accordance with the relevant guidelines and regulations.

### Intervention - duration and probiotic administration

All probiotic strains were administered in form of suspension (10⁹ CFU/ml) once a day for 9 weeks. Control animals received saline. Dose was estimated on the basis of changes in miRNA profile *in vitro.* The number of animals per intervention group was 8-10 individuals.

### Tissue preparation and miRNA assay

Tissue homogenates were prepared according to the following steps:
- weighing the hippocampi and cerebellum (the isolated structures were stored at -80 °C, the weighing was carried out in the shortest possible time to minimize the risk of complete tissue thawing, and the brain structures were kept on ice after weighing);
- preparation of homogenates; isolated hippocampi were sonicated (20 seconds, 50% amplitude) (Sonics Vibra Cell sonicator) in a cold, previously prepared buffer comprising 5M guanidine hydrochloride and 50 mM Tris; sonication was performed in a buffer volume equal to 8 times the tissue weight.
- prepared homogenates were shaken for 3-4 hours on an orbital shaker; then the samples were diluted 10 times using cold BSAT-PBS buffer (5% BSA; 0.03% Tween20 and 1x PBS Carl Roth) containing a cocktail of protease inhibitors (Halt Protease & Phosphatase Inhibitor Single-Use Cocktail (100x), Thermo Scientific);
- homogenates were centrifuged at 16,000 x g, 20 min, 4 °C and the supernatant was transferred to new Eppendorf tubes and frozen at -80 °C. RNA isolation and miRNA assay has been performed as described in 'Materials and methods - Experimental example 3' above.

### Experimental example 5 - Open field results for strains in AD model

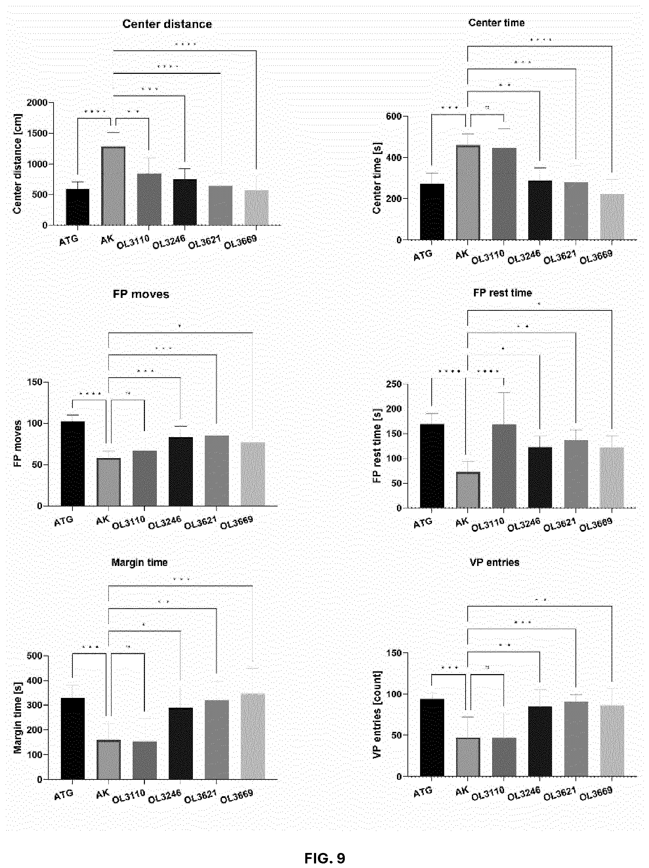

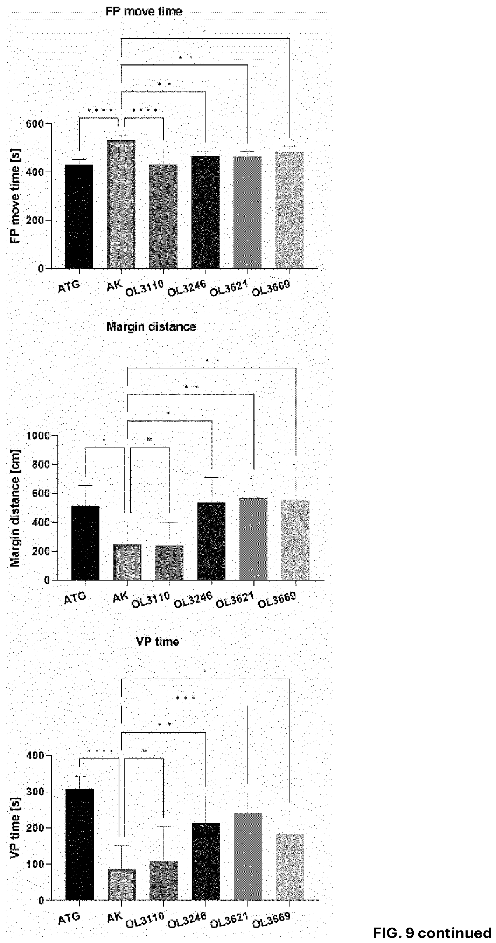

After 60 days of administration of tested probiotic strains significant improvement in open field results were observed. AD-mice spent more time in center and horizontal plane, covered more distance in center than mice without mutation. Reverse association for rest of tested parameters - higher values for Control and lowered for AD-mice (Fig. 9) were also observed. All tested strains were able to significantly improve or completely restore tested parameters.

Fig. 9 shows the open field results. Each plot represents data for a specific parameter (center distance, center time, FP moves, FP rest time, margin time, VP entries, FP move time, margin distance, VP time, described in detail below). In this experiment genetic background (ATG) was used as a healthy control, genetically modified mice (AK) as a control with disease. Statistical analysis was performed using ordinary one-way ANOVA with Dunnett correction against AK group. (ns - statistically insignificant (P ≥ 0.05); * - statistically significant (P ranging from 0.01 to 0.05); ** - statistically significant (P ranging from 0.001 to 0.01); *** - statistically significant (P < 0.001); **** - statistically significant (P < 0.0001).

### Experimental example 6 - Open field results for STRAINS in PD model

Parkinson's disease (PD) is a neurodegenerative disease characterized by akinesia, rigidity, resting tremor, and postural instabilities. In addition, neuropsychiatric, perceptual and cognitive deficits are increasingly recognized as non-motor manifestations of Parkinson's disease (Carbon et al., 2010; Carbon & Eidelberg, 2006). Open field test, as murine tool to verify cognitive deficits is widely used in PD research (Aniszewska et al., 2022; Su et al., 2018).

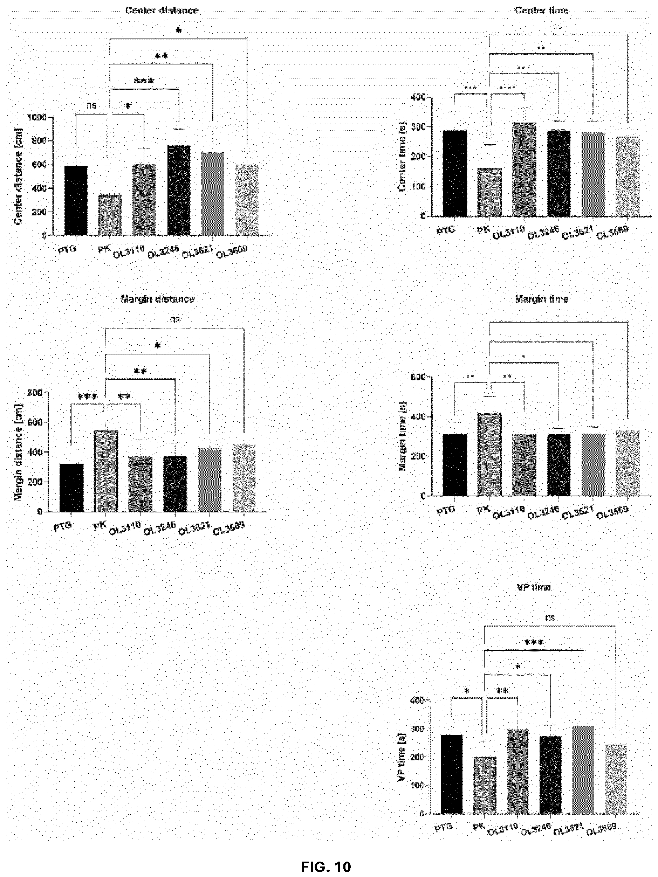

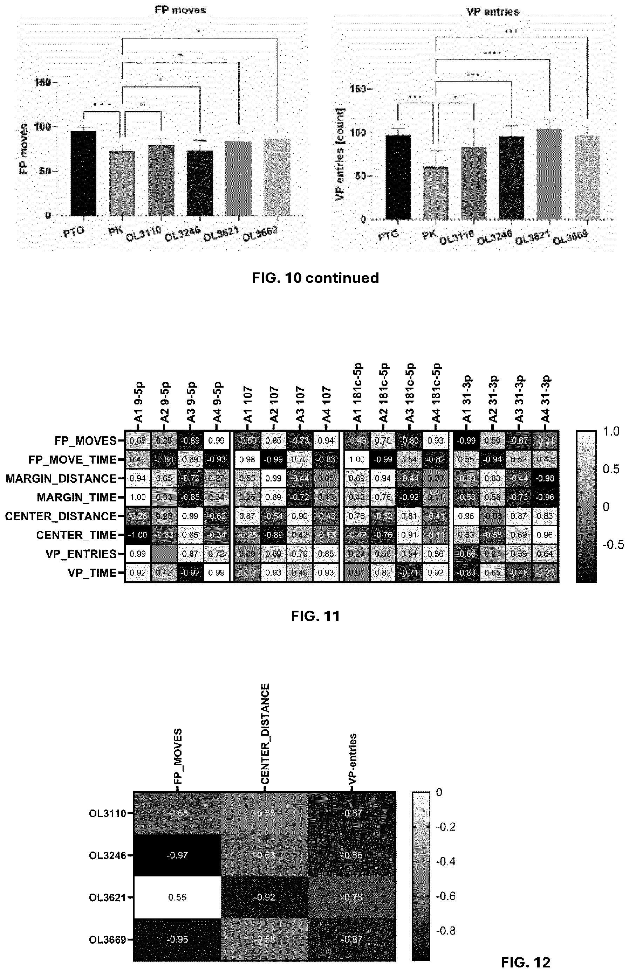

Fig. 10 shows the open field results. Each plot represents data for a specific parameter (mentioned in Example 5 above, and discussed in detail below). In this experiment genetic background (PTG), was used as a healthy control, genetically modified mice (PK) as a control with disease. Statistical analysis was performed using ordinary one-way ANOVA with Dunnett correction against the PK group. (ns - statistically insignificant (P ≥ 0.05); * - statistically significant (P ranging from 0.01 to 0.05); ** - statistically significant (P ranging from 0.001 to 0.01); *** - statistically significant (P < 0.001); **** - statistically significant (P < 0.0001).

### Materials and methods (Experimental example 5 and 6)

### Open field test

In the open field test, the animal's general locomotor and exploratory activity as well as anxiety behavior were examined. Open field test is able to measure cognitive performance and its widely used for verification of cognitive deficits and effectiveness of future therapeutics (Ferreira et al., 2022; Seibenhener & Wooten, 2015). Briefly, the test was performed using the TruScan Coulborn device. It is a 26 cm x 26 cm cage in which the animal is placed and its activity is measured for 10 minutes by infrared-light sensors.

The following parameters were analyzed:
FP_MOVES - parameter describing the number of movements in the horizontal plane
FP_MOVE_TIME - parameter describing the duration of movements in the horizontal plane [s]
FP_REST_TIME - parameter describing the duration of immobility in the horizontal plane [s]
FP_DISTANCE - parameter describing the distance covered by the animal in the horizontal plane [cm]
FP_VELOCITY - parameter describing the speed at which the animal moved in the horizontal plane [cm/s]
MARGIN_DISTANCE - distance covered in the field edge area [cm]
MARGIN_TIME - time spent on the edge of the field [s]
CENTER_DISTANCE - distance covered in the central part of the field [cm]
CENTER_TIME - time spent in the central part of the field [s]
VP_ENTRIES - number of elevations of the animal's body, i.e. entering the vertical plane
VP_TIME - total time spent in the raised position, i.e. entering the vertical plane [s]

### Experimental example 7 - association between miRNA up/downregulation and change in cognitive parameters in open field

Based on the experimental results (see Experimental example 5 and 6), it can be concluded that administration of selected probiotic strains effectively ameliorated cognitive deficits present in both AD and PD-mice. Mice fed with probiotics explored more center of the cage (parameters: center_time, center distance) and also vertical space. They also spent less time in margins and exhibited similar behavior as healthy controls. These results clearly suggest that mice with mutations (AD and PD-mice) have reduced ability to explore and learn new environment and also have more pronounced anxiety and deterioration than healthy controls and more importantly - than probiotic-fed groups, which proves the effectiveness of selected strains in improvement of cognitive parameters in AD and PD murine models.

To determine the association between observed cognitive- and mood-improvement a matrix of correlations was prepared, showing that probiotics and their constituents directly modulate miRNAs, eventually lading to restoring "normal" performance in PD and AD-mice (Experimental example 6).

Fig. 11 shows Pearson's Correlation matrix between open field parameters and miRNA changes in AD mouse model.

To improve the visibility in fig. 11 the strain ID's were changed as follows: A1-OL3110, A2-OL3246, A3-OL3621, A4-3669.

Fig. 12. Pearson's Correlation matrix between open field parameters and miRNA changes in PD mouse model.

### Experimental example 8 - Rota-rod results as proxy of neuromotor abilities in mice model of PD disease

It is generally difficult to discriminate motor and cognitive aspects in behavioral tests of Parkinson's disease patients, because impaired movement can influence of the entire behavioral performance. Due to this fact, the present Inventors decided to verify how animals with enhanced alpha-synuclein aggregation performed in rota-rod test.

Among several behavioral tests that measure motor performance, the rota-rod test is a suitable test for evaluation of cerebellar deficits in rodents. Additionally, some studies detected impairment of rota-rod performance in dopamine-depleted mice and rats (Shiotsuki et al., 2010), suggesting that this test is suitable even in case of neurodegeneration in other brain regions.

In the described study the Inventors observed reduced time spent on rod in case of PD-mice, which indicated motor deficits in that group compared to healthy animals (PTG). All treatment groups were able to restore murine neuromotor abilities at various degrees. Strains OL3110 and OL3246 restored neuromotor abilities to the similar degree as in healthy animals. For OL3621 and OL3669 improvement was smaller, but significant when compared to PD-mice .

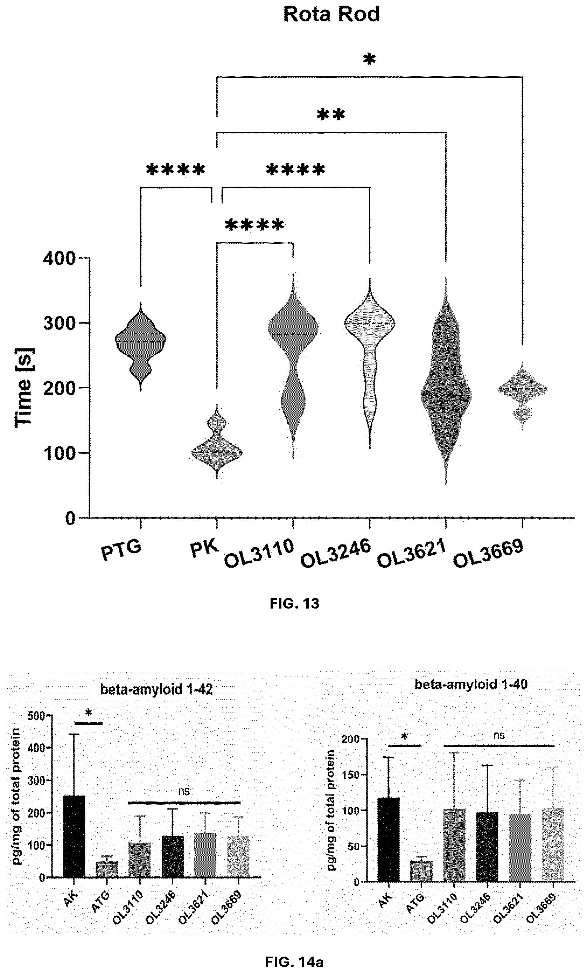

Fig. 13 shows Rota-rod results. In this experiment genetic background (PTG), was used as a healthy control, genetically modified mice (PK) as a control with disease. Statistical analysis was performed using ordinary one-way ANOVA with Dunnett correction against PK group. (ns - statistically insignificant (P ≥ 0.05); * - statistically significant (P ranging from 0.01 to 0.05); ** - statistically significant (P ranging from 0.001 to 0.01); *** - statistically significant (P < 0.001); **** - statistically significant (P < 0.0001).

### Experimental example 8 - Material and methods

### Rota-Rod test

Rota-rod test was performed using standard device and procedures (Shiotsuki et al., 2010). Briefly, each tested animal was placed on rotating rod and after 10s of familiarization the speed of rotation increased and time spent on rod was measured. Minimal time: 10s, maximal time: 300s; all measurements were performed in triplicate. All other necessary means and procedures i.e. animals and housing, were described above (see experimental example 4).

### Experimental example 9 - association between miRNA and Rota-rod results

Similar as in case of open field test, a correlation was made with miRNA 376-3p, which was dysregulated both in *in vitro* and *in vivo* experiment. A significant negative correlation was obtained for all tested strains (see Table 2), meaning that the lower the miRNA 376 expression was the better neuromotor abilities were, clearly indicating the connection between miRNA 376 as a regulatory compound and tested novel probiotic strains. Probiotic strains manipulate levels of miRNA 376, resulting in overall improved neuromotor abilities.

**Table 2. Pearson correlation results between miRNA 376-3p levels and time spent on Rotarod of PD mice model. OL3110, OL3246, OL3621, OL3669.**

| | 376-3p | | | |
|---|---|---|---|---|
| | OL3110 | OL3246 | OL3621 | OL3669 |
| Pearson r value | -0.69 | -0.80 | -0.64 | -0.93 |

### Experimental example 10 - positive abilities of tested strains are not associated with previously described influence on inflammatory status, BDNF, beta-amyloid or alpha-synuclein levels.

Both KO(knockout)-mice models expressed significant changes in its marker proteins - beta-amyloids (AD-mice) and alpha-synuclein (PD-mice). Contrary to state of knowledge, the present Inventors did not observe any significant (neither positive nor negative) influence of tested strains on the mentioned proteins levels. Similar results were observed with respect to serum cytokines and brain derived neurotrophic factor (BDNF), indicating the lack of association between positive cognitive results and changes in beta-amyloids, alpha-synuclein and BDNF or in immune status.

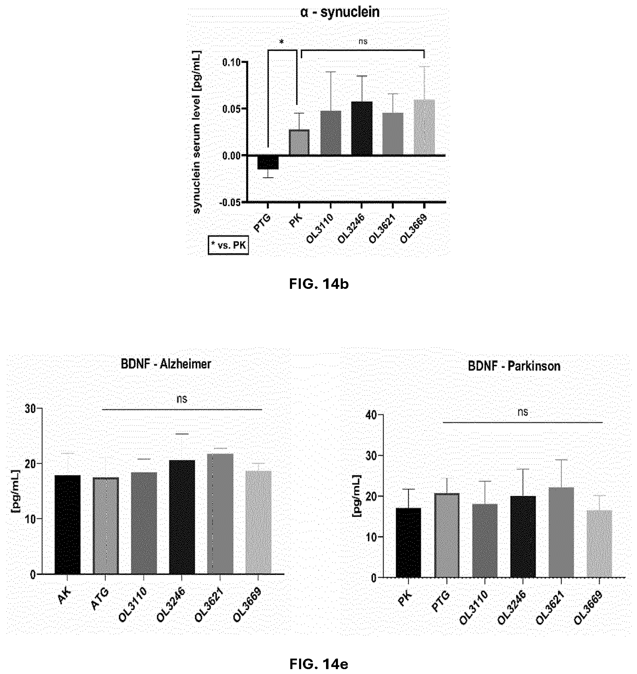

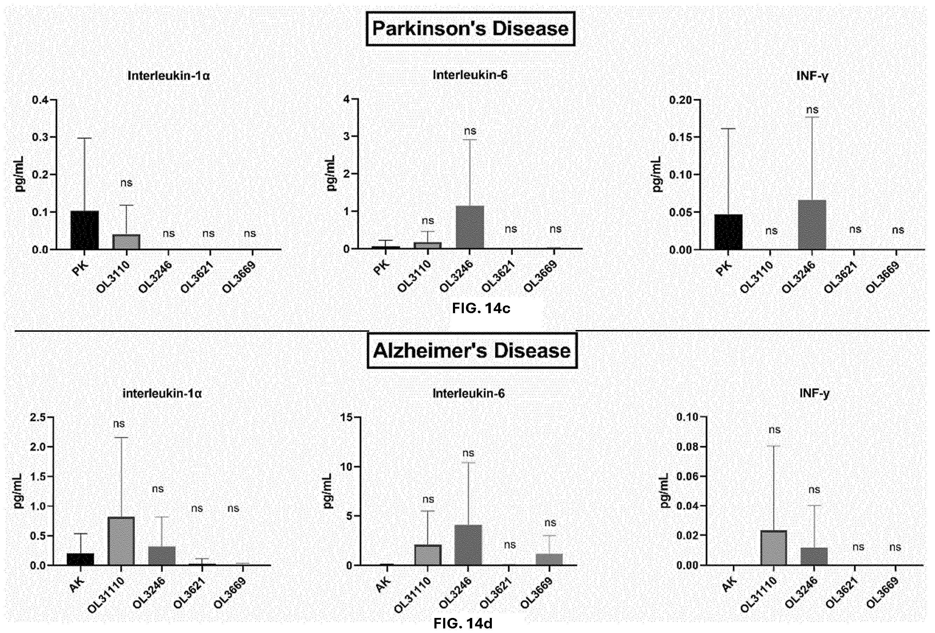

Fig. 14 show the results of biochemical assays of previously described molecular markers associated with AD and PD. All analysis were performed on blood or tissue samples originating from *in vivo* experiment (see: Experimental examples 4-9). More specifically, fig. 14a show the results of beta-amyloid 1-40 and 1-42 deposition in hippocampus. Tissue homogenates were prepared as described in Experimental example 4 (Materials and methods). Both assays were performed using ELISA kits according to manufacturer's recommendations (ThermoFisher cat. No. KMB3441, ThermoFisher cat. No. KMB3481). Fig. 14b shows alpha-synuclein levels in serum of PD-mice. This analysis was performed according to manufacturer's recommendations of Mouse SNCa (Synuclein, Alpha) ELISA Kit (Fine Test cat. no. EM1369). Fig. 14c and 14d show cytokine profile in serum of AD and PD-mice, respectively. The analysis was performed in accordance to manufacturer's recommendations using flow cytometry and CBA (cytometric bead array) flex-set kit (BD-Biosciences) containing antibodies against: IL-1alfa (cat. No. 560157), IL-1beta (cat. No. 560232), IL-2 (cat. No. 558297), IL-6 (cat. No. 558301), IL-10 (cat. No. 558300), IL-12 (cat. No. 558303), MCP-1 (cat.no. 558342), TNF (cat. No. 558299), INF-gamma (cat. No. 558296). Graphs present cytokines, the level of which was above detection threshold in more than one sample. Fig. 14e shows BDNF levels in serum of PD and AD-mice. The assay was performed according to manufacturer's recommendations using Total BDNF Quantikine ELISA Kit (kat. No. DBNT00, R&D Systems).

### Experimental example 11 - capsules with strain(s) in form of lyophilized powder without additives

| **INGREDIENTS** | **mg/ capsule** | **weight percentage %** |
|---|---|---|
| Maltodextrin (filling agent) | 385.500 | 77.100% |
| Strain(s) in the form of lyophilized bacteria ie. OL3621 (1*10¹¹ CFU/g) | 103.500 | 20.700% |
| Magnesium stearate (glidant) | 6.500 | 1.300% |
| Silica (anticaking agent) | 4.500 | 0.900% |
| Capsule filling mass | 500.000 | 100.000% |

| | | |
|---|---|---|
| Capsule shells: cellulose, pullulan, or gelatin, enteric-coated | | |

### Bibliography:

Aniszewska, A., Bergström, J., Ingelsson, M., & Ekmark-Lewén, S. (2022). Modeling Parkinson's disease-related symptoms in alpha-synuclein overexpressing mice. Brain and Behavior, 12(7), 1-16. https://doi.org/10.1002/brb3.2628
Aziz, R. K., Bartels, D., Best, A., DeJongh, M., Disz, T., Edwards, R. A., Formsma, K., Gerdes, S., Glass, E. M., Kubal, M., Meyer, F., Olsen, G. J., Olson, R., Osterman, A. L., Overbeek, R. A., McNeil, L. K., Paarmann, D., Paczian, T., Parrello, B., ... Zagnitko, O. (2008). The RAST Server: Rapid annotations using subsystems technology. BMC Genomics, 9, 1-15. https://doi.org/10.1186/1471-2164-9-75
Bartel, D. P. (2004). MicroRNAs: Genomics, Biogenesis, Mechanism, and Function. Cell, 116(2), 281-297. https://doi.org/10.1016/S0092-8674(04)00045-5
Carbon, M., & Eidelberg, D. (2006). Functional imaging of sequence learning in Parkinson's disease. Journal of the Neurological Sciences, 248(1-2), 72-77. https://doi.org/10.1016/j.jns.2006.05.005
Carbon, M., Reetz, K., Ghilardi, M. F., Dhawan, V., & Eidelberg, D. (2010). Longitudinal Changes in Brain Activity during Sequence Learning. Neurobiology of Disease, 37(2), 1-15. https://doi.org/10.1016/j.nbd.2009.10.025.Early
Chen, J., Zeng, B., Li, W., Zhou, C., Fan, S., Cheng, K., Zeng, L., Zheng, P., Fang, L., Wei, H., & Xie, P. (2017). Effects of gut microbiota on the microRNA and mRNA expression in the hippocampus of mice. Behavioural Brain Research, 322, 34-41. https://doi.org/https://doi.org/10.1016/j.bbr.2017.01.021
Chopin, A., Chopin, M. C., Moillo-Batt, A., & Langella, P. (1984). Two plasmid-determined restriction and modification systems in Streptococcus lactis. Plasmid, 11(3), 260-263. https://doi.org/10.1016/0147-619x(84)90033-7
Costantini, L., Molinari, R., Farinon, B., & Merendino, N. (2017). Impact of omega-3 fatty acids on the gut microbiota. In International Journal of Molecular Sciences. https://doi.org/10.3390/ijms18122645
Davey, H. M., & Kell, D. B. (1996). Flow cytometry and cell sorting of heterogeneous microbial populations: the importance of single-cell analyses. Microbiological Reviews, 60(4), 641-696. https://doi.org/10.1128/mr.60.4.641-696.1996
David P. Bartel. (2009). MicroRNA Target Recognition and Regulatory Functions. Cell, 136(2), 215-233. https://doi.org/10.1016/j.cell.2009.01.002.MicroRNA
Davoodvandi, A., Marzban, H., Goleij, P., Sahebkar, A., Morshedi, K., Rezaei, S., Mahjoubin-Tehran, M., Tarrahimofrad, H., Hamblin, M. R., & Mirzaei, H. (2021). Effects of therapeutic probiotics on modulation of microRNAs. Cell Communication and Signaling, 19(1), 1-22. https://doi.org/10.1186/s12964-020-00668-w
de Rijke, T. J., Doting, M. H. E., van Hemert, S., De Deyn, P. P., van Munster, B. C., Harmsen, H. J. M., & Sommer, I. E. C. (2022). A Systematic Review on the Effects of Different Types of Probiotics in Animal Alzheimer's Disease Studies. Frontiers in Psychiatry, 13(April), 1-17. https://doi.org/10.3389/fpsyt.2022.879491
DeVries, L., Horstman, C., Fossell, M., & Carlson, C. (2021). Ingestion of Bifidobacterium longum changes miRNA levels in the brains of mice. PLoS ONE, 16(4 April), 1-11. https://doi.org/10.1371/journal.pone.0249817
Dikeocha, I. J., Al-Kabsi, A. M., Hussin, S., & Alshawsh, M. A. (2020). Role of probiotics in patients with colorectal cancer: A systematic review protocol of randomised controlled trial studies. BMJ Open, 10(8).https://doi.org/10.1136/bmjopen-2020-038128
Douros, A., Ante, Z., Fallone, C. A., Azoulay, L., Renoux, C., Suissa, S., & Brassard, P. (2024). Clinically apparent Helicobacter pylori infection and the risk of incident Alzheimer's disease: A population-based nested case-control study. Alzheimer's and Dementia, 20(3), 1716-1724. https://doi.org/10.1002/alz.13561
DP, B. (2018). Metazoan MicroRNAs. Cell, 173(1), 20-51. https://doi.org/10.1016/j.cell.2018.03.006.Metazoan
Ferreira, J. S., Leite Junior, J. B., de Mello Bastos, J. M., Samuels, R. I., Carey, R. J., & Carrera, M. P. (2022). A new method to study learning and memory using spontaneous locomotor activity in an open-field arena. Journal of Neuroscience Methods, 366, 109429. https://doi.org/10.1016/j.jneumeth.2021.109429
Garcia-Gonzalez, N., Bottacini, F., van Sinderen, D., Gahan, C. G. M., & Corsetti, A. (2022). Comparative Genomics of Lactiplantibacillus plantarum: Insights Into Probiotic Markers in Strains Isolated From the Human Gastrointestinal Tract and Fermented Foods. Frontiers in Microbiology, 13(May).https://doi.org/10.3389/fmicb.2022.854266
Ghosh, T. S., Shanahan, F., & O'Toole, P. W. (2022). The gut microbiome as a modulator of healthy ageing. Nature Reviews Gastroenterology and Hepatology, 19(9), 565-584. https://doi.org/10.1038/s41575-022-00605-x
Heiland, T., Zeitschel, U., Puchades, M. A., Kuhn, P. H., Lichtenthaler, S. F., Bjaalie, J. G., Hartlage-Rübsamen, M., Roßner, S., & Höfling, C. (2019). Defined astrocytic expression of human amyloid precursor protein in Tg2576 mouse brain. Glia, 67(2), 393-403. https://doi.org/10.1002/glia.23550
Hill, C., Guarner, F., Reid, G., Gibson, G. R., Merenstein, D. J., Pot, B., Morelli, L., Canani, R. B., Flint, H. J., Salminen, S., Calder, P. C., & Sanders, M. E. (2014). Expert consensus document: The international scientific association for probiotics and prebiotics consensus statement on the scope and appropriate use of the term probiotic. Nature Reviews Gastroenterology and Hepatology, 11(8), 506-514. https://doi.org/10.1038/nrgastro.2014.66
Jäger, R., Mohr, A. E., Carpenter, K. C., Kerksick, C. M., Purpura, M., Moussa, A., Townsend, J. R., Lamprecht, M., West, N. P., Black, K., Gleeson, M., Pyne, D. B., Wells, S. D., Arent, S. M., Smith-Ryan, A. E., Kreider, R. B., Campbell, B. I., Bannock, L., Scheiman, J., ... Antonio, J. (2019). International Society of Sports Nutrition Position Stand: Probiotics. Journal of the International Society of Sports Nutrition, 16(1), 1-44. https://doi.org/10.1186/s12970-019-0329-0
Jastrz 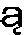 b, R., Graczyk, D., & Siedlecki, P. (2021). Molecular and Cellular Mechanisms Influenced by Postbiotics*.*
Jin, X., Dong, W., Chang, K., Yan, Y., & Liu, X. (2024). Efficacy of probiotic supplements on Parkinson's disease: A systematic review and meta-analysis. Complementary Therapies in Medicine, 82(May), 103045. https://doi.org/10.1016/j.ctim.2024.103045
Johnson, J. S., Spakowicz, D. J., Hong, B. Y., Petersen, L. M., Demkowicz, P., Chen, L., Leopold, S. R., Hanson, B. M., Agresta, H. O., Gerstein, M., Sodergren, E., & Weinstock, G. M. (2019). Evaluation of 16S rRNA gene sequencing for species and strain-level microbiome analysis. Nature Communications, 10(1), 1-11. https://doi.org/10.1038/s41467-019-13036-1
Jonas, S., & Izaurralde, E. (2015). Towards a molecular understanding of microRNA-mediated gene silencing. Nature Reviews Genetics, 16(7), 421-433. https://doi.org/10.1038/nrg3965
Le, D. T. L., Tran, T. L., Duviau, M. P., Meyrand, M., Guérardel, Y., Castelain, M., Loubière, P., Chapot-Chartier, M. P., Dague, E., & Mercier-Bonin, M. (2013). Unraveling the role of surface mucus-binding protein and pili in muco-adhesion of Lactococcus lactis. PLoS ONE, 8(11). https://doi.org/10.1371/journal.pone.0079850
Leta, V., Ray Chaudhuri, K., Milner, O., Chung-Faye, G., Metta, V., Pariante, C. M., & Borsini, A. (2021). Neurogenic and anti-inflammatory effects of probiotics in Parkinson's disease: A systematic review of preclinical and clinical evidence. Brain, Behavior, and Immunity, 98, 59-73. https://doi.org/10.1016/j.bbi.2021.07.026
Liang, D., Liu, H., Jin, R., Feng, R., Wang, J., Qin, C., Zhang, R., Chen, Y., Zhang, J., Teng, J., Tang, B., Ding, X., & Wang, X. (2023). Escherichia coli triggers α-synuclein pathology in the LRRK2 transgenic mouse model of PD. Gut Microbes, 15(2). https://doi.org/10.1080/19490976.2023.2276296
Matthews, K. A., Xu, W., Gaglioti, A. H., Holt, J. B., Croft, J. B., Mack, D., & McGuire, L. C. (2019). Racial and ethnic estimates of Alzheimer's disease and related dementias in the United States (2015-2060) in adults aged ≥65 years. Alzheimer's and Dementia, 15(1), 17-24. https://doi.org/10.1016/j.jalz.2018.06.3063
Mishra, V., Yadav, D., Solanki, K. S., Koul, B., & Song, M. (2024). A Review on the Protective Effects of Probiotics against Alzheimer's Disease. Biology, 13(1), 1-21. https://doi.org/10.3390/biology13010008
Mukherjee, S., Mez, J., Trittschuh, E. H., Saykin, A. J., Gibbons, L. E., Fardo, D. W., Wessels, M., Bauman, J., Moore, M., Choi, S.-E., Gross, A. L., Rich, J., Louden, D. K. N., Sanders, R. E., Grabowski, T. J., Bird, T. D., McCurry, S. M., Snitz, B. E., Kamboh, M. I., ... Crane, P. K. (2018). Genetic data and cognitively defined late-onset Alzheimer's disease subgroups. Molecular Psychiatry, 1. https://doi.org/10.1038/s41380-018-0298-8
Nataraj, B. H., Ali, S. A., Behare, P. V., & Yadav, H. (2020). Postbiotics-parabiotics: The new horizons in microbial biotherapy and functional foods. Microbial Cell Factories, 19(1), 1-22. https://doi.org/10.1186/s12934-020-01426-w
Olson, R. D., Assaf, R., Brettin, T., Conrad, N., Cucinell, C., Davis, J. J., Dempsey, D. M., Dickerman, A., Dietrich, E. M., Kenyon, R. W., Kuscuoglu, M., Lefkowitz, E. J., Lu, J., Machi, D., Macken, C., Mao, C., Niewiadomska, A., Nguyen, M., Olsen, G. J., ... Stevens, R. L. (2023). Introducing the Bacterial and Viral Bioinformatics Resource Center (BV-BRC): a resource combining PATRIC, IRD and ViPR. Nucleic Acids Research, 51(1 D), D678-D689. https://doi.org/10.1093/nar/gkac1003
Ondov, B. D., Treangen, T. J., Melsted, P., Mallonee, A. B., Bergman, N. H., Koren, S., & Phillippy, A. M. (2016). Mash: Fast genome and metagenome distance estimation using MinHash. Genome Biology, 17(1), 1-14. https://doi.org/10.1186/s13059-016-0997-x
Paumier, K. L., Sukoff Rizzo, S. J., Berger, Z., Chen, Y., Gonzales, C., Kaftan, E., Li, L., Lotarski, S., Monaghan, M., Shen, W., Stolyar, P., Vasilyev, D., Zaleska, M., D. Hirst, W., & Dunlop, J. (2013). Behavioral Characterization of A53T Mice Reveals Early and Late Stage Deficits Related to Parkinson's Disease. PLoS ONE, 8(8).https://doi.org/10.1371/journal.pone.0070274
Prasansuklab, A., Sukjamnong, S., Theerasri, A., Hu, V. W., Sarachana, T., & Tencomnao, T. (2023). Transcriptomic analysis of glutamate-induced HT22 neurotoxicity as a model for screening anti-Alzheimer's drugs. Scientific Reports, 13(1), 1-13. https://doi.org/10.1038/s41598-023-34183-y
Radziwill-Bienkowska, J. M., Le, D. T. L., Szczesny, P., Duviau, M. P., Aleksandrzak-Piekarczyk, T., Loubière, P., Mercier-Bonin, M., Bardowski, J. K., & Kowalczyk, M. (2016). Adhesion of the genome-sequenced Lactococcus lactis subsp. cremoris IBB477 strain is mediated by specific molecular determinants. Applied Microbiology and Biotechnology, 100(22), 9605-9617. https://doi.org/10.1007/s00253-016-7813-0
Romano, S., Sawa, G. M., Bedarf, J. R., Charles, I. G., Hildebrand, F., & Narbad, A. (2021). Meta-analysis of the Parkinson's disease gut microbiome suggests alterations linked to intestinal inflammation. Npj Parkinson's Disease, 7(1).https://doi.org/10.1038/s41531-021-00156-z
Sa 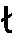 ański, P., Kowalczyk, M., Bardowski, J. K., & Szczepankowska, A. K. (2022). Health-Promoting Nature of Lactococcus lactis IBB109 and Lactococcus lactis IBB417 Strains Exhibiting Proliferation Inhibition and Stimulation of Interleukin-18 Expression in Colorectal Cancer Cells. Frontiers in Microbiology, 13(May).https://doi.org/10.3389/fmicb.2022.822912
Saleena, L. A. K., Teo, M. Y. M., How, Y. H., In, L. L. A., & Pui, L. P. (2023). Immunomodulatory action of Lactococcus lactis. Journal of Bioscience and Bioengineering, 135(1), 1-9. https://doi.org/10.1016/J.JBIOSC.2022.10.010
Salminen, S., Collado, M. C., Endo, A., Hill, C., Lebeer, S., Quigley, E. M. M., Sanders, M. E., Shamir, R., Swann, J. R., Szajewska, H., & Vinderola, G. (2021). The International Scientific Association of Probiotics and Prebiotics (ISAPP) consensus statement on the definition and scope of postbiotics. Nature Reviews Gastroenterology and Hepatology, 18(9), 649-667. https://doi.org/10.1038/s41575-021-00440-6
Sanderson, T. H., Raghunayakula, S., & Kumar, R. (2015). Release of mitochondrial Opa1 following oxidative stress in HT22 cells. Molecular and Cellular Neuroscience, 64, 116-122. https://doi.org/10.1016/j.mcn.2014.12.007
Seibenhener, M. L., & Wooten, M. C. (2015). Use of the open field maze to measure locomotor and anxiety-like behavior in mice. Journal of Visualized Experiments, 96, 1-6. https://doi.org/10.3791/52434
Shiotsuki, H., Yoshimi, K., Shimo, Y., Funayama, M., Takamatsu, Y., Ikeda, K., Takahashi, R., Kitazawa, S., & Hattori, N. (2010). A rotarod test for evaluation of motor skill learning. Journal of Neuroscience Methods, 189(2), 180-185. https://doi.org/https://doi.org/10.1016/j.jneumeth.2010.03.026
Su, R. J., Zhen, J. L., Wang, W., Zhang, J. L., Zheng, Y., & Wang, X. M. (2018). Time-course behavioral features are correlated with Parkinson's disease-associated pathology in a 6-hydroxydopamine hemiparkinsonian rat model. Molecular Medicine Reports, 17(2), 3356-3363. https://doi.org/10.3892/mmr.2017.8277
Sun, D., Gao, W., Hu, H., & Zhou, S. (2022). Why 90% of clinical drug development fails and how to improve it? Acta Pharmaceutica Sinica B, 12(7), 3049-3062. https://doi.org/10.1016/j.apsb.2022.02.002
Wei, W., Wang, Z. Y., Ma, L. N., Zhang, T. T., Cao, Y., & Li, H. (2020). MicroRNAs in Alzheimer's Disease: Function and Potential Applications as Diagnostic Biomarkers. Frontiers in Molecular Neuroscience, 13(August), 1-16. https://doi.org/10.3389/fnmol.2020.00160
Westerman, M. A., Cooper-Blacketer, D., Mariash, A., Kotilinek, L., Kawarabayashi, T., Younkin, L. H., Carlson, G. A., Younkin, S. G., & Ashe, K. H. (2002). The relationship between Aβ and memory in the Tg2576 mouse model of Alzheimer's disease. Journal of Neuroscience, 22(5), 1858-1867. https://doi.org/10.1523/jneurosci.22-05-01858.2002
Xue, X., Cao, A. T., Cao, X., Yao, S., Carlsen, E. D., Soong, L., Liu, C. G., Liu, X., Liu, Z., Duck, L. W., Elson, C. O., & Cong, Y. (2014). Downregulation of microRNA-107 in intestinal CD11c+ myeloid cells in response to microbiota and proinflammatory cytokines increases IL-23p19 expression. European Journal of Immunology, 44(3), 673-682. https://doi.org/10.1002/eji.201343717
Zhang, H., & Cai, Y. (2014). Lactic acid bacteria: Fundamentals and practice. In Lactic Acid Bacteria: Fundamentals and Practice. https://doi.org/10.1007/978-94-017-8841-0
Zhu, G., Zhao, J., Zhang, H., Wang, G., & Chen, W. (2023). Gut Microbiota and its Metabolites: Bridge of Dietary Nutrients and Alzheimer's Disease. Advances in Nutrition, 14(4), 819-839. https://doi.org/10.1016/j.advnut.2023.04.005

## Claims

1. A probiotic bacterial strain capable of direct manipulation of miRNA levels in a mammalian brain, said strain being selected from:
*Lactococcus lactis* OL3669, deposited with the KPD under the accession number 1757,
*Lactiplantibacillus plantarum* OL3246, deposited with the KPD under the accession number 1760,
*Lacticaseibacillus paracasei* OL3110, deposited with the KPD under the accession number 1759, and
*Leuconostoc mesenteroides* OL3621, deposited with the KPD under the accession number 1758.

2. The probiotic bacterial strain defined in claim 1 for use in alleviating at least one syndrome of a neurodegenerative disease in a mammalian patient.

3. A composition for alleviating at least one syndrome of a neurodegenerative disease in a human patient, said composition comprising at least one probiotic bacterial strain defined in claim 1 in an effective amount and/or a bacteria cell culture supernatant obtained from a culture of at least one probiotic bacterial strain defined in claim 1 and/or at least one isolated component of the bacteria cell culture supernatant.

4. The composition according to claim 3, wherein the neurodegenerative disease is selected from Alzheimer's disease (AD), Parkinson's disease (PD) and other diseases negatively affecting neuronal system and being associated and/or modulated by miRNA 107, 31, 181, 376 and 9.

5. The composition according to claim 3 or 4, wherein said at least one probiotic bacterial strain defined in claim 1 contains live bacteria, dead bacteria or both live and dead bacteria.

6. The composition according to claim 5, wherein the concentration of live or dead bacteria ranges from 10³ to 10¹¹ CFU per gram of dry weight of the composition.

7. The composition according to claim 5, wherein the bacteria of at least one probiotic bacterial strain defined in claim 1 and/or the bacteria cell supernatant and/or the isolated component thereof are in lyophilized form.

8. The composition according to any of claims 3-6, wherein said at least one probiotic bacterial strain defined in claim 1 is form of a culture suspension.

9. The composition according to any of claims 3-8 in a solid form selected from capsule, tablet and powder or granulate for forming a solution or suspension.

10. The composition according to any of claims 3-8 in a liquid form selected from solution, suspension and emulsion.

11. The composition according to claim 10, wherein the concentration of live or dead bacteria ranges from 10³ to 10¹¹ CFU per millilitre of the liquid composition.

12. The composition according to any of claims 10-11, wherein the bacteria cell culture supernatant concentration in the liquid composition ranges from 2 to 35% v/v.

13. The composition according to any of claims 3-12, wherein the bacteria are contained in microcapsules.

14. The composition according to claim 13, wherein the microcapsules contain live bacteria as well as auxiliary substances selected from lipids, phospholipids, proteins, polysaccharides or a mixture thereof.

15. The composition according to any of claims 3-14, further comprising at least one additional ingredient selected from pharmaceutically acceptable excipients, aromatizing agents, carriers and dietary supplements such as vitamins, minerals, herbal extracts, carbohydrates, lipids and proteins.
